# EUROPEAN PATENT APPLICATION

(11) **EP 2 371 352 A2**
(43) Date of publication of application: **05.10.2011**
(21) Application number: 11002490.8
(22) Date of filing: 01.02.2002
(51) Int. Cl.: A61K 9/00, A61L 27/34, A61L 27/54

(54) **Devices including protein matrix materials and methods of making and using thereof**

(30) Priority: 03.08.2001 US 922418
(62) Divisional of application: 02714808.9
(71) Applicant: Gel-Del Technologies, Inc., St. Paul, MN 55108 (US)
(72) Inventor: Masters, David, Hastings, Minnesota 55033 (US)
(74) Representative: Arends, William Gerrit

(57) **Abstract**

A device comprising biocompatible protein matrix materials and their method of preparation and use, wherein the device is a protein matrix diagnostic array, a protein matrix coated implantable medical device or an electromatrix device. The biocompatible protein matrix materials comprise one or more biocompatible proteins and one or more biocompatible solvents. The protein matrix diagnostic array can be used in a method of determining pharmacologically active agent to protein interaction.

## Description

### Governmental Rights

At least a portion of the research described in this application was supported in part by United States Governmental funding in the form of NIH Grant No. 5R01GM51917. The United States Government has certain rights in the invention.

### Field of the Invention

The present invention relates to devices including a protein matrix material and the methods of making and using such devices. More specifically the present invention relates to protein matrix devices that may be utilized for various medical applications including, but not limited to, current (magnetic and electric) released drug delivery devices for the controlled release of pharmacologically active agents, electromatrix devices (e.g. antennae, leads, chips, wires, etc), coatings for implantable medical devices (e.g. Micro-Electronics Minaturization Systems (MEMS), pacemakers, etc.) and imaging and diagnostic devices. Furthermore, the present invention relates to devices including a protein matrix made by forming a film comprising one or more biocompatible protein materials and one or more biocompatible solvents. The film may also optionally include one or more pharmacologically active agents and/or one or more conductive materials. The film is then partially dried, rolled or otherwise shaped, and then compressed to form the desired protein matrix device. During the rolling or shaping of the film, one or more conductive materials, and/or one or more implantable devices may be placed into the film and thereby compressed to form a coating around the conductive materials, and/or implantable devices.

### Background of the Invention

Protein materials are generally present in the tissues of many biological species. Therefore, the development of medical devices that utilize protein materials, which mimic and/or are biocompatible with the host tissue, have been pursued as desirable devices due to their acceptance and incorporation into such tissue. For example the utilization of protein materials to prepare drug delivery devices for the controlled release of pharmacologically active agents, electromatrix devices (e.g. antennae, leads, chips, wires, etc), coatings for implantable medical devices (e.g. Micro-Electronics Minaturization Systems (MEMS), pacemakers, etc.), imaging and diagnostic devices and other similar types of medical devices have been perceived as being valuable products due to their biocompatibility and ability to function with biological materials.

The use of dried protein, gelatins, hydrogels and/or synthetic polymers have previously been used as components for the preparation of devices for drug delivery, coatings for implantable medical devices, imaging and diagnostic devices and the like. However, many of these previously developed devices do not offer sufficient strength, stability, support and/or biocompatibility when administered to tissue environments that contain high solvent content, such as the tissue environment of the human body. Furthermore, the features of such medical devices that additionally incorporate pharmacologically active agents often provide an ineffective and uncontrollable release of such agents, thereby not providing an optimal device for controlled drug delivery.

A concern and disadvantage of such devices is the rapid dissolving or degradation of the device upon entry into an aqueous or high solvent environment. For example, gelatins and compressed dry proteins tend to rapidly disintegrate and/or lose their form when placed in an aqueous environment. Therefore, many dried or gelatin type devices do not provide optimal drug delivery and/or structural and durability characteristics.

Gelatins often contain large amounts of water or other liquid that makes the structure fragile, non-rigid and unstable. It is also noted that the proteins of gelatins usually denature during preparation caused by heating, thereby reducing or eliminating the beneficial characteristics of the protein. Alternatively, dried protein devices are often very rigid, tend to be brittle and are extremely susceptible to disintegration upon contact with solvents. The deficiencies gelatins and dried matrices have with regards to rapid degradation and structure make such devices less than optimal for the controlled release of pharmacologically active agents, or for operating as the biocompatible coatings for implantable medical devices or imaging and diagnostic devices.

Hydrogel-forming polymeric materials, in particular, have been found to be useful in the formulation of medical devices, such as drug delivery devices. See, e.g., Lee, J. Controlled Release, 2, 277 (1985). Hydrogel-forming polymers are polymers that are capable of absorbing a substantial amount of water to form elastic or inelastic gels. Many non-toxic hydrogel-forming polymers are known and are easy to formulate. Furthermore, medical devices incorporating hydrogel-forming polymers offer the flexibility of being capable of being implantable in liquid or gelled form. Once implanted, the hydrogel forming polymer absorbs water and swells. The release of a pharmacologically active agent incorporated into the device takes place through this gelled matrix via a diffusion mechanism.

However, many hydrogels, although biocompatible, are not biodegradable or are not capable of being remodeled and incorporated into the host tissue. Furthermore, most medical devices comprising hydrogels require the use of undesirable organic solvents for their manufacture. Residual amounts of such solvents potentially remain in the medical device, where they cause solvent-induced toxicity in surrounding tissues or cause structural or pharmacological degradation to the pharmacologically active agents incorporated within the medical device. Finally, implanted medical devices that incorporate pharmacologically active agents in general, and such implanted medical devices comprising hydrogel-forming polymers in particular, oftentimes provide suboptimal release characteristics of the drug(s) incorporated therein. That is, typically, the release of pharmacologically active agents from an implanted medical device that includes pharmacologically active agent(s) is irregular, e.g., there is an initial burst period when the drug is released primarily from the surface of the device, followed by a second period during which little or no drug is released, and a third period during which most of the remainder of the drug is released or alternatively, the drug is released in one large burst.

It would be desirable to provide a protein matrix device that would biocompatibly degrade and/or resorb into the host tissue for which it is administered. Alternatively, it would be desirable to provide a protein matrix medical device that can be incorporated and remodeled by the host tissue to remain in the tissue and provide a prolonged intended function of the device. Furthermore, it would be desirable to provide improved implantable protein matrix medical devices capable of being biodegradable and resorbable or alternatively capable of being incorporated and remodeled into the host tissue, such that removal of the device is not necessary while also optionally capable of sustained, controlled local delivery of pharmacologically active agents when implanted. It would further be desirable to control the rate of delivery from such devices to avoid possible side effects associated with irregular delivery, e.g., high drug concentration induced tissue toxicity. Finally, it would be advantageous if such devices could be manufactured with biocompatible proteins and solvents so that the potential for residual solvent toxicity, inflammation and immunogenicity is reduced.

### Summary of the Invention

The present invention relates to devices including and utilizing a protein matrix material and the methods of making and using such devices. Embodiments of the present invention may include, but are not limited to, current released drug delivery devices for the controlled release of pharmacologically active agents, electromatrix devices (e.g. antennae, leads, chips, wires, etc), coatings for implantable medical devices (e.g. Micro-Electronics Minaturization Systems (MEMS), pacemakers, etc.), imaging and diagnostic devices and other similar types of medical devices.

Furthermore, the present invention relates to a method of making devices that include the protein matrix material by forming a coatable composition comprising one or more biocompatible protein materials, one or more biocompatible solvents and optionally one or more pharmacologically active agents and/or one or more conductive materials. The coatable composition may also include additional polymeric materials and/or therapeutic entities that would provide additional beneficial characteristics or features to the protein matrix such as enhanced durability and drug release characteristics. Once the coatable composition is formed, it is then coated so as to form a film on a surface or substrate (preferably the film has a substantially planar body having opposed major surfaces and preferably having a thickness between the major surfaces of from about 0.01 millimeters to about 5 millimeters). Next, the film is at least partially dried until it is cohesive, and then formed (rolled, folded, accordion-pleated, crumpled, or otherwise shaped) into a cohesive body having a surface area less than that of the film. The film may also be folded, rolled, crumpled or otherwise manipulated to encompass, cover or mask one or more conductive materials or one or more implantable medical devices. The cohesive body, optionally including the conductive materials and/or medical devices, is then compressed to provide the desired protein matrix device in accordance with the present invention.

The cohesive body, which may include the pharmacologically active agent, conductive material, implantable medical device and/or other similar material, is compressed to limit bulk biocompatible solvent, such as bulk or trapped water (i.e., iceberg water). The elimination of the bulk biocompatible solvent by compressing enhances the strength and durability of the matrix by initiating, stimulating and forcing additional intramolecular and intermolecular attraction between the biocompatible solvent molecules, such as hydrogen bonding activity, and also initiates, stimulates and forces intramolecular and intermolecular activity between the protein molecules, the biocompatible solvent molecules and the optional pharmacologically active agents, conductive materials implantable medical devices and/or imaging and diagnostic devices.

The above described process has many advantages if one or more pharmacologically active agents are incorporated into the protein matrix material. For example, the controlled release characteristics of the protein matrix provides for a higher amount of pharmacologically active agent(s) that may be incorporated into the matrix. Additionally, the pharmacologically active agent(s) is/are substantially homogeneously distributed throughout the protein matrix material. This homogenous distribution provides for a more systematic and consistent release of the pharmacologically active agent(s). As a result, the release characteristics of the pharmacologically active agent from the protein matrix device are enhanced.

As previously suggested, embodiments of the protein matrix devices produced utilizing the method of the present invention are capable of the sustainable, controllable local delivery of pharmacologically active agent(s), while also providing the advantage of being capable of being safely resorbed and remodeled by the host tissue. The resorbable and remodeling characteristics of various embodiments of the present invention eliminates the need for the removal of the protein matrix device from the patient once the pharmacologically active agent(s) have been completely delivered from the matrix.

Additionally, other embodiments of the present invention may be produced to enhance the stability and durability of the protein matrix materials by incorporating one or more additional polymeric materials into the protein matrix or by treating the protein matrix material with a reagent. For example, the protein matrix material may be partially or totally treated with a reagent, such as glutaraldehyde, to create crosslinking of the protein fibers in the matrix. The crosslinking of the protein material may be utilized to produce a biocompatible device that has a desired function, form or shape, such as an antennae, chip or graft, and additionally may retain its form without completely resorbing or degrading into the patient or until the matrix has been incorporated and/or remodeled into the host tissue. Also, the crosslinking of a protein matrix device may be utilized to prevent attachment of materials such as other proteins or cells to the matrix where it is not desired. Examples of protein matrix devices that would benefit from such nondegradable characteristics include, but are not limited to, coatings for implantable medical devices, imaging and diagnostic devices and other devices that need a biocompatible sustaining structure to remain in the patient.

As previously mentioned, such devices may further include one or more pharmacologically agents. The nondegradable protein matrix device would still retain the systematic release of the pharmacological active agents, thereby diffusing out of the device rather than releasing upon degradation of the protein matrix material.

Whether the protein matrix device is intended to be entirely resorbable or not, the method of making the protein matrix devices is generally the same. In describing the method more specifically, the method comprises the steps of preparing a coatable composition comprising one or more biocompatible protein materials, one or more biocompatible solvents and optionally one or more pharmacologically active agents and/or one or more conductive materials. Additional biodegradable polymeric materials may be added in the preparation of the coatable composition to provide optimum features desired for the particular protein matrix device being prepared. For example, polyanhydride may be added to the protein matrix to inhibit the absorption of physiological body fluids, thereby limiting the amount of fluids interacting with the conductive material and/or medical device. Also, addition of polyanhydride may act to slow the diffusion and/or resorption of the protein matrix and/or pharmacological active agent from the protein matrix device. The coatable composition is then coated to form a film and partially dried until the coated film can be formed into a cohesive body, e.g., preferably until the film has a solvent content of from about 50% to about 70%. The film is then shaped into a cohesive body, e.g., rolled, folded, accordion-pleated, crumpled, or otherwise shaped into a cylinder or shaped into a ball, cube and the like, preferably with a surface area less than that of the film. The cohesive body is then compressed to remove as much of the solvent as possible so that the compressed body remains cohesive, but without removing so much solvent that the compressed body becomes brittle or otherwise lacks cohesiveness. Typically, the resulting protein matrix device has a solvent content of from about 10% to about 60%, preferably from about 30% to about 50%. During the rolling or shaping of the film, one or more conductive materials, and/or one or more implantable devices may be placed into the film and thereby compressed to form a coating around the conductive materials, and/or implantable devices. In addition, if desired, the compressed body may next be treated with a crosslinking reagent, such as glutaraldehyde to form a compressed body that has additional structural and nonresorbable features.

As previously suggested, by coating the aforementioned components into a film, partially drying the film, forming the film into a cohesive body and subsequently compressing the cohesive body, a protein matrix device, which includes one or more pharmacologically active agents, has a substantially homogeneous distribution of the pharmacologically active agent(s). Due to this substantially homogeneous distribution, the protein matrix devices of the present invention that include one or more pharmacologically active agents provide a sustainable and controllable release of the pharmacologically active agent(s). Furthermore, the method of the present invention utilizes biocompatible, and if selected, resorbable and biodegradable, protein materials. As a result, protein matrix devices formed in accordance with the method of the present invention may include the benefit of remaining in the patient indefinitely or simply resorbing and/or degrading into the tissue surrounding it. Finally, since the protein matrix material is biocompatible, any solvent remaining in the protein matrix device after the manufacture thereof presents a reduced, if not substantially eliminated, risk of producing undesirable side effects when implanted into a patient.

The biocompatible protein material incorporated into a device in accordance with the present invention generally comprises one or more biocompatible proteins, which preferably are a water-absorbing, biocompatible protein. Additionally, the biocompatible protein may be synthetic, genetically engineered or natural. In various embodiments of the present invention, the genetically engineered protein material comprises silklike blocks and elastinlike blocks. The biocompatible solvent may be water, dimethyl sulfoxide (DMSO), ethanol, an oil, combinations of these, or the like. Preferably, the biocompatible solvent comprises water. As previously indicated, the protein matrix device can incorporate any desired pharmacologically active agent or even a second drug delivery device, e.g., corticosteroids, opioid analgesics, neurotoxins, local anesthetics, vesicles, lipospheres, microspheres, nanospheres, enzymes, combinations of these, and the like. The conductive material may comprise any material that can carry an electrical current, such as ceramics, copper, iron, stainless steel or any other metal, alloy or material that is utilized for transmission of current. Finally, the implantable medical devices that may be included in the present invention include but are not limited to pacemakers, implantable defibrillators, heart pumps, MEMS such as Peltier devices, catheters, or any other medical implants.

As previously mentioned, the protein matrix devices may be administered for systemic delivery of pharmacologically active agents. Preferably, the therapeutic response effected is an analgesic response, an anti-inflammatory response, an anesthetic response, a response preventative of an immunogenic response, an anti-coagulatory response, antithrombogenic response, a genetic response, a protein assembly response, an antibacterial response, a vaccination response, combinations of these, and the like. As used herein, unless stated otherwise, all percentages are percentages based upon the total mass of the composition being described, e.g., 100% is total.

### Brief Description of the Figures

The above mentioned and other advantages of the present invention, and the manner of attaining them, will become more apparent and the invention itself will be better understood by reference to the following description of the embodiments of the invention taken in conjunction with the accompanying drawing, wherein:
Figure 1 is a schematic illustration, in partial cross-sectional view, of a compression molding device that may be used in the method of the present invention in a configuration prior to compression;
Figure 2 is a schematic illustration, in partial cross-sectional view, of a compression molding device that may be used in the method of the present invention in a configuration during compression;
Figure 3 is a schematic illustration, in partial cross-sectional view, of a compression molding device that may be used in the method of the present invention in a configuration during ejection; and
Figure 4 depicts an embodiment of a current release drug device of the present invention including conductive materials distributed throughout the matrix;
Figure 5 depicts an embodiment of a current release drug delivery device of the present invention wherein conductive materials are positioned around the circumference of the cylinder;
Figure 6A depicts an embodiment of a protein matrix device of the present invention including a conductive material in the shape of a tube;
Figure 6B depicts an embodiment of a protein matrix device of the present invention in the shape of a cylinder further including a conductive material distributed throughout the matrix;
Figure 7 depicts an embodiment of an antennae of the present invention;
Figure 8 depicts an embodiment of a lead of the present invention;
Figure 9 depicts various embodiments of an encapsulated mesh or screen device ;
Figure 10 depicts an embodiment of an electrode of the present invention;
Figure 11 depicts an embodiment of an implantable medical device including a protein matrix coating;
Figure 12 depicts an embodiment of a diagnostic protein matrix array plate of the present invention.

### Detailed Description of the Invention

The embodiments of the invention described below are not intended to be exhaustive or to limit the invention to the precise forms disclosed in the following detailed description. Rather, the embodiments are chosen and described so that others skilled in the art may appreciate and understand the principles and practices of the present invention. The present invention relates to devices including protein matrix materials and the methods of making and using such protein matrix devices. More specifically, the method of preparation of the present invention involves preparing a coatable composition comprising one or more biocompatible protein materials, one or more biocompatible solvents and optionally one or more pharmacologically active agents and/or one or more conductive materials. It is noted that additional polymeric materials and/or therapeutic entities may be included in the coatable composition to provide various beneficial features such as strength, elasticity, structure and/or any other desirable characteristics. Once the coatable composition is formed it is then coated to a surface or substrate to form a film that is subsequently partially dried, formed into a cohesive body, and then compressed to provide a protein matrix device in accordance with the present invention. During the rolling or shaping of the film into a cohesive body, one or more conductive materials, and/or one or more implantable devices may be placed into the film and thereby compressed to form a coating around the conductive materials, and/or implantable devices.

While not wishing to be bound by any theory, it is believed that by preparing a coatable composition from the aforementioned components, coating this composition to form a film that is subsequently partially dried, and then forming the film into a cohesive body, a relatively homogeneous distribution of the components is obtained in the cohesive body. Furthermore, when the film has dried enough so as to be cohesive unto itself, e.g., to a solvent content from about 50% to about 70%, subsequently formed into a cohesive body and then compressed many, if not all, of any distribution anomalies are removed or resolved. Therefore, when the protein matrix device includes a pharmacologically active agent and /or a conductive material, the distribution of the pharmacologically active agent is rendered substantially homogenous throughout the resulting protein matrix device.

In addition, the removal of such distribution anomalies also includes the removal of bulk or trapped biocompatible solvent, such as aqueous solutions, i.e. bulk water (i.e., iceberg water) from the matrix. For example, in aqueous solutions, proteins bind some of the water molecules very firmly and others are either very loosely bound or form islands of water molecules between loops of folded peptide chains. Because the water molecules in such an island are thought to be oriented as in ice, which is crystalline water, the islands of water in proteins are called icebergs. Furthermore, water molecules may also form bridges between the carbonyl (C=O) and imino (NH) groups of adjacent peptide chains, resulting in structures similar to those of a pleated sheet (β-sheets) but with a water molecule in the position of the hydrogen bonds of that configuration. Generally, the amount of water bound to one gram of a globular protein in solution varies from 0.2 to 0.5 grams. Much larger amounts of water are mechanically immobilized between the elongated peptide chains of fibrous proteins, such as gelatin. For example, one gram of gelatin can immobilize at room temperature 25 to 30 grams of water. It is noted that other biocompatible solvents may also interact with protein molecules to effect intra- and inter-molecular forces upon compression. The compression of the cohesive body removes bulk solvent from the resulting protein matrix device.

The protein matrix devices of the present invention traps biocompatible solvent molecules, such as water molecules, and forces them to interact with the protein to produce a protein-water matrix with natural physical, biological and chemical characteristics. The compression of the cohesive body eliminates the islands of water or bulk water resulting in a strengthened protein matrix structure. Furthermore, the elimination of bulk water enhances the homogenous characteristics of the protein matrix by reducing the pooling of water and spacing of the protein molecules, pharmacologically active agent molecules and conductive material molecules. Upon compression of the cohesive body, the remaining water molecules are forced to interact with most to all protein molecules, pharmacologically active agent molecules and conductive material molecules and thereby adds strength, structure and stability to the protein matrix. The compression forces out most of the non-structural bulk water (immobilized water) from the matrix. As previously suggested, the bulk water is extra water that is only loosely bound to the matrix. Additionally, the water that interacts with the protein molecules of the protein matrix reduces and/or prevents the protein from denaturing during compression and facilitates the protein binding with the water through intra- and inter-molecular forces (i.e., ionic, dipole-dipole such as hydrogen bonding, London dispersion, hydrophobic, etc.). The enhanced binding characteristics of the protein matrix further inhibits the loss of non-bulk solvent molecules that interact with protein molecules. Experiments have indicated that a protein matrix dries to 25-45% water during overnight drying processes that would normally dry over 100 times that same amount of water if it were not in the matrix.

Furthermore, the resulting protein matrix device preferably has as little solvent as possible while still being cohesive and possessing the desired features relevant to the device's function, e.g., preferably a solvent content of from about 10% to about 60%, more preferably a solvent content of from about 30% to about 50%. It is found that when a protein matrix device of the present invention includes a pharmacologically active agent, the partial drying of the film to form a cohesive body and subsequent compressing of the cohesive body, forces more solvent out of the body, thereby producing a resulting protein matrix device that has a significantly higher concentration of pharmacologically active agent relative to other components of the device than is obtainable in protein devices produced by other methods. As a result of the substantially uniform dispersion of a greater concentration of pharmacologically active agent, a sustained, controlled release of the pharmacologically active agent is achieved, while reducing the initial high concentration effects that can be associated with other devices that include pharmacologically active agents or bolus injections of pharmacologically active agents.

Reducing the solvent content has the additional effect that the resulting protein matrix device is more structurally sound, easy to handle, and thus, easy to insert or implant. Upon insertion, the cells of the tissue contacting the implanted protein matrix holds the protein matrix device substantially in the desired location. Alternatively, embodiments of the protein matrix may be held in the desired location by tissue contact, pressure, sutures, adhesives and/or tissue folds or creases. Embodiments of the protein matrix device may biodegrade and resorbs over time or retain their structural integrity.

To form the coatable composition, the biocompatible protein material(s), the biocompatible solvent(s), and optionally the pharmacologically active agent(s) and/or conductive materials may be combined in any manner; for example simple mixing. It is noted that one or more additional polymeric materials and/or therapeutic entities may be added to the coatable composition during the combination step to provide additional desirable characteristics to the coatable composition. For example, the components may simply be combined in one step, or alternatively, the biocompatible protein materials may be dissolved and/or suspended in a biocompatible solvent and an additional protein material and/or the pharmacologically active agent may be dissolved and/or suspended in the same or another biocompatible solvent and then the resulting two solutions mixed.

Once prepared, the coatable composition may be coated onto any suitable surface from which it may be released after drying by any suitable method. Examples of suitable coating techniques include spin coating, gravure coating, flow coating, spray coating, coating with a brush or roller, screen printing, knife coating, curtain coating, slide curtain coating, extrusion, squeegee coating, and the like. The coated film (preferably having a substantially planar body having opposed major surfaces) is desirably thin enough so as to be capable of drying within a reasonable amount of time and also thin enough so that the film can be formed into a cohesive body comprising a substantially homogeneous dispersion of the components of the coatable composition. For example, a thinner film will tend to form a more homogeneous cohesive body when the film is formed into the shape of a cylinder. A typical coated film of the coatable composition have a thickness in the range of from about 0.01 millimeters to about 5 millimeters, more preferably from about 0.05 millimeters to about 2 millimeters.

Initially, when the film is first coated, it is likely to be non-cohesive, fluidly-flowable, and/or non self-supporting. Thus, the coated film is preferably dried sufficiently so that it becomes cohesive, i.e., the film preferably sticks to itself rather than other materials. The film may simply be allowed to dry at room temperature, or alternatively, may be dried under vacuum, conditions of mild heating, i.e., heating to a temperature of from about 25°C to about 50°C, or conditions of mild cooling, i.e. cooling to a temperature of from about 0°C to about 10°C. When utilizing heat to dry the film, care should be taken to avoid denaturation or structural degradation of the pharmacologically active agent incorporated therein.

The specific solvent content at which the film becomes cohesive unto itself will depend on the individual components incorporated into the coatable composition. Generally, films that have too high of a solvent content will not be cohesive. Films that have too low of a solvent content will tend to crack, shatter, or otherwise break apart upon efforts to form them into a cohesive body. With these considerations in mind, the solvent content of a partially dried film will preferably be from about 20% to about 80%, more preferably from about 30% to about 65% and most preferably from about 35% to about 50%.

Once the film is capable of forming a cohesive body, such a cohesive body may be formed by any of a number of methods. For example, the film may be rolled, folded, accordion-pleated, crumpled, or otherwise shaped such that the resulting cohesive body has a surface area that is less than that of the coated film. For example the film can be shaped into a cylinder, a cube, a sphere or the like. Preferably, the cohesive body is formed by rolling the coated film to form a cylinder.

Once so formed, the cohesive body is compressed to form a protein matrix device in accordance with the present invention. Any manually or automatically operable mechanical, pneumatic, hydraulic, or electrical molding device capable of subjecting the cohesive body to pressure and provides for removal of the bulk solvent is suitable for use in the method of the present invention. In the production of various embodiments of the present invention, a molding device may be utilized that is capable of applying a pressure of from about 100 pounds per square inch (psi) to about 100,000 psi for a time period of from about 2 seconds to about 48 hours. Preferably, the molding device used in the method of the present invention will be capable of applying a pressure of from about 1000 psi to about 30,000 psi for a time period of from about 10 seconds to about 60 minutes. More preferably, the molding device used in the method of the present invention will be capable of applying a pressure of from about 3,000 psi to about 25,000 psi for a time period of from about one minute to about ten minutes.

Compression molding devices suitable for use in the practice of the method of the present invention are generally known. Suitable devices may be manufactured by a number of vendors according to provided specifications, such as desirable pressure, desired materials for formulation, desired pressure source, desired size of the moldable and resulting molded device, and the like. For example, Garni Engineering, located in Mississauga, Ontario manufactures compression molding devices to specifications provided by the customer. Additionally, many compression molding devices are commercially available.

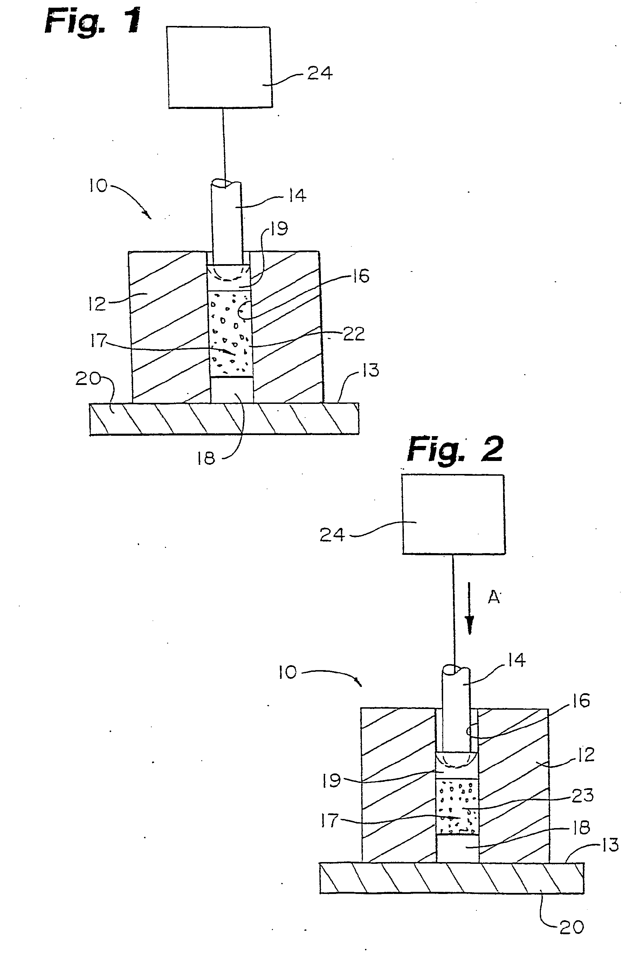

An embodiment of a compression molding device 10 suitable for use in the method of the present invention is schematically shown in Figure 1. Compression molding device 10 is equipped with a mold body 12 in which cohesive body 22 can be subjected to pressure in order to compress and mold the cohesive body 22 into a protein matrix device in accordance with the present invention. Mold body 12 is shown supported in position on a base plate 20. More specifically, mold body 12 has provided therein a cavity 16 that preferably extends all the way through mold body 12. Within the cavity 16 a molding chamber 17 can be defines into which a cohesive body in accordance with the present invention may be inserted. The molding chamber 17 may be configured in any shape and size depending upon the shape and size of the protein matrix device. For example, the chamber may take the shape or form of a tube, heart valve, cylinder or any other desired shape. The cavity 16 may comprise a bore of any shape that may be machined, formed, cast or otherwise provided into the mold body 12. The compression molding device may optionally include one or more apertures of approximately .004 to .0001 inches for biocompatible solvent to escape the chamber 17 during compression of the cohesive body. An inner insert 18 is preferably slidably fit within cavity 16 to be positioned against one surface 13 of the base plate 20 to define the molding chamber 17 and support to cohesive body 22 when positioned within the molding chamber 17. The insert 18 may be any shape that is desired for molding the protein matrix device. For example the insert 18 may be a solid cylindrical mandrel that can form the lumen of a tube or vessel. The insert 18 is thus fixed with respect to the mold body 12 to define the inner extent of the molding chamber 17. An outer insert 19 is also preferably provided to be slidable within the cavity 16.

Outer insert 19 is used to close the molding chamber 17 of cavity 16 after the inner insert 18 and the cohesive body 22 are provided in that order within the cavity 16. The inner and outer inserts 18 and 19, respectively, can be the same or different from one another, but both are preferably slidably movable within the cavity 16. The inner and outer inserts 18 and 19, respectively, are configured to create the desired form or shape of the protein matrix device. Additionally, the inserts 18 and 19 may be shaped similarly to the shape of the cavity 16 to slide therein and are sized to effectively prevent the material of the cohesive body 22 to pass between the inserts 18 and 19 and the walls of cavity 16 when the cohesive body 22 is compressed as described below. However, the sizing may be such that moisture, such as bulk solvent, can escape between the outer edges of one or both inserts 18 and 19 and the surface walls of the cavity 16 from the cohesive body 22 during compression. Otherwise, other conventional or developed means can be provided to permit moisture, such as bulk solvent, to escape from the mold cavity during compression. For example, small openings could pass through one or both of the inserts 18 and 19 or mold body 12 which may also include one-way valve devices. Insert 18 may be eliminated so that surface 13 of base plate 20 defines the lower constraint to molding chamber 17. However, the use of insert 18 is beneficial, in that its presence facilitates easy removal of the cohesive body 22 after compression (described below) and provides a sufficiently hard surface against which the cohesive body 22 can be compressed. Moreover, by utilizing a series of differently sized and/or shaped inner inserts 18, the volume of the molding chamber can be varied, or different end features may be provided to the cohesive body 22. Outer inserts 19 can likewise be varied.

Outer insert 19 is also positioned to be advanced within cavity 16 or retracted from cavity 16 by a plunger 14. Preferably, the contacting surfaces of outer insert 19 and plunger 14 provide a cooperating alignment structure so that pressure can be evenly applied to the cohesive body 22. The plunger 14 may comprise a part of, or may be operatively connected with a pressure generation mechanism 24 that has the ability to apply pressure of the type and force necessary to achieve the results of the present invention. Conventional or developed technologies are contemplated, such as using mechanical, hydraulic, pneumatic, electrical, or other systems. Such systems can be manually or automatically operable.

Plunger 14 operates independently of mold body 12 and is operationally coupled to the pressure generation mechanism 24. Pressure generation mechanism 24 may be any pressure source capable of applying from about 100 psi to about 100,000 psi for a time period of from about 2 seconds to about 48 hours, preferably capable of applying from about 1000 psi to about 30,000 psi for a time period of from about 10 seconds to about 60 minutes, and more preferably, capable of applying a pressure of from about 3000 psi to about 25,000 psi for a time period of from about 1 minute to about 10 minutes. Preferably, plunger 14 is formulated of a material capable of translating substantially all of the pressure applied by pressure generation mechanism 24 to cohesive body 22.

Mold body 12 may be fabricated from any material capable of withstanding the - pressure to be applied from pressure generation mechanism 24, e.g., high density polyethylene, Teflon®, steel, stainless steel, titanium, brass, copper, combinations of these and the like. Desirably, mold body 12 is fabricated from a material that provides low surface friction to inserts 18 and 19 and cohesive body 22. Alternatively, surfaces defining the cavity 16 may be coated with a low friction material, e.g., Teflon®, to provide such low surface friction. Due to its relatively low cost, sufficient strength and surface friction characteristics, mold body 12 is desirably fabricated from brass. Cavity 16, extending substantially through mold body 12, may be of any shape and configuration, as determined by the desired configuration of the resulting, compressed protein matrix devices. In one embodiment, cavity 16 is cylindrical. However, the shape of the cavity 16 can be configured to accommodate the shape and size of the resulting, compressed protein matrix device. As above, inserts 18 and 19 preferably fit within cavity 16 in a manner that allows moisture to escape from mold body 12, and so that inserts 18 and 19 may be easily inserted into and removed from cavity 16. Furthermore, it is preferred that inserts 18 and 19 fit within cavity 16 in a manner that provides adequate support and containment for cohesive body 22, so that, upon compression, the material of cohesive body 22 does not escape cavity 16 in a manner that would produce irregularly shaped edges on the resulting protein matrix device.

According to one procedure for using compression molding device 10 to carry out the method of the present invention, the mold body 12 is positioned as shown in Figure 1 on the base plate 20, which itself may be supported in any manner. Then, an inner insert 18 is placed into cavity 16 followed by a cohesive body 22 to be compressed and an outer insert 19 as shown. As previously noted, the cohesive body 22 may also include the insertion of one or more conductive materials or one or more medical devices (not shown). Plunger 14 is then positioned so as to be in driving engagement with outer insert 19. Then, as schematically illustrated in Figure 2, the pressure generation mechanism 24 is activated to move plunger 14 in the direction of arrow A to reduce the volume of the molding cavity 17 to make a compressed cohesive body 23. Pressure generation mechanism 24 applies sufficient pressure, i.e., from about 100 psi to about 100,000 psi for a time period of from about 2 seconds to about 48 hours, to plunger 14, insert 19 and cohesive body 22 against the inner insert 18, thereby driving moisture from and compressing cohesive body 22 into a protein matrix device in accordance with the present invention.

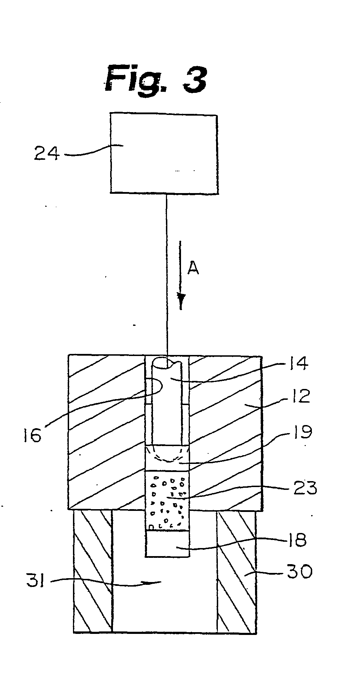

As shown in Figure 3, the compressed cohesive body 23 can then be ejected from the mold body 12 along with inserts 18 and 19 by positioning the mold body 12 on a support spacer 30 and further advancing the plunger 14 in the direction of arrow A by the pressure generation mechanism 24. Generally, base plate 20 is separated from the mold body 12 when ejecting the protein matrix device and inserts 18 and 19. The support spacer 30 is preferably shaped and dimensioned to provide an open volume 31 for the compressed cohesive body 23 to be easily removed. That is, when the plunger 14 is sufficiently advanced, the insert 18 and compressed cohesive body 23 can fall into the open volume 31 within the support spacer 30. After completion, the plunger 14 can be fully retracted so that the compression molding device 10 can be reconfigured for a next operation.

Any biocompatible protein material may be utilized in the protein matrix devices and corresponding methods of the present invention. Preferably, any such material will at least be water-compatible, and more preferably will be water-absorbing or hydrogel forming. Furthermore, one or more biocompatible protein materials may be incorporated into the protein matrix device of the present invention and may desirably be selected based upon their biocompatible and/or degradation properties. The combination of more than one biocompatible protein can be utilized to mimic the environment in which the device is to be administered, optimize the biofunctional characteristics, such as cell attachment and growth, nonimmuno-response reaction and/or alter the release characteristics, or duration of an included pharmacologically active agent, if a pharmacologically active agent is to be included in the device.

The biocompatible protein material may comprise one or more biocompatible synthetic protein, genetically-engineered protein, natural protein or any combination thereof. In many embodiments of the present invention, the biocompatible protein material comprises a water-absorbing, biocompatible protein. In various embodiments of the present invention, the utilization of a water-absorbing biocompatible protein provides the advantage that, not only will the protein matrix device be biodegradable, but also resorbable and/or able to be remodeled by the host tissue. That is, that the metabolites of the degradation of the water-absorbing biodegradable protein may be reused by the patient's body rather than excreted. In other embodiments that do not degrade or resorb the water absorbing material provides enhanced biocompatible characteristics since the device is generally administered to environments that contain water.

As previously mentioned, the biocompatible protein utilized may either be naturally occurring, synthetic or genetically engineered. Naturally occurring protein that may be utilized in the protein matrix device of the present invention include, but are not limited to elastin, collagen, albumin, keratin, fibronectin, silk, silk fibroin, actin, myosin, fibrinogen, thrombin, aprotinin, antithrombin III and any other biocompatible natural protein. It is noted that combinations of natural proteins may be utilized to optimize desirable characteristics of the resulting protein matrix, such as strength, degradability, resorption, etc. Inasmuch as heterogeneity in molecular weight, sequence and stereochemistry can influence the function of a protein in a protein matrix device, in some embodiments of the present invention synthetic or genetically engineered proteins are preferred in that a higher degree of control can be exercised over these parameters.

Synthetic proteins are generally prepared by chemical synthesis utilizing techniques known in the art. Examples of such synthetic proteins include but are not limited to natural protein made synthetically and collagen linked GAGS like collagen-heparin, collagen-chondroitin and the like. Also, individual proteins may be chemically combined with one or more other proteins of the same or different type to produce a dimer, trimmer or other multimer. A simple advantage of having a larger protein molecule is that it will make interconnections with other protein molecules to create a stronger matrix that is less susceptible to dissolving in aqueous solutions.

Additional, protein molecules can also be chemically combined to any other chemical so that the chemical does not release from the matrix. In this way, the chemical entity can provide surface modifications to the matrix or structural contributions to the matrix to produce specific characteristics. The surface modifications can enhance and/or facilitate cell attachment depending on the chemical substance or the cell type. Furthermore, the structural modifications can be used to facilitate or impede dissolution, enzymatic degradation or dissolution of the matrix.

Synthetic biocompatible materials may be cross-linked, linked, bonded or chemically and/or physically linked to pharmacological active agents and utilized alone or in combination with other biocompatible proteins to form the cohesive body. Examples of such cohesive body materials include, but are not limited to heparin-protein, heparin-polymer, chondroitin-protein, chondroitin-polymer, heparin-cellulose, heparin-alginate, heparin-polylactide, GAGs-collagen, heparin-collagen.

Specific examples of a particularly preferred genetically engineered proteins for use in the protein matrix devices of the present invention is that commercially available under the nomenclature "ELP", "SLP", "CLP", "SLP", "SLPF" and "SELP" from Protein Polymer Technologies, Inc. San Diego, CA. ELP's, SLP's, CLP's, SLPL's, SLPF's and SELP's are families of genetically engineered protein polymers consisting of silklike blocks, elastinlike blocks, collagenlike blocks, lamininlike blocks, fibronectinlike blocks and the combination of silklike and elastinlike blocks, respectively. The ELP's, SLP's, CLP's, SLPL's, SLPF's and SELP's are produced in various block lengths and compositional ratios. Generally, blocks include groups of repeating amino acids making up a peptide sequence that occurs in a protein. Genetically engineered proteins are qualitatively distinguished from sequential polypeptides found in nature in that the length of their block repeats can be greater (up to several hundred amino acids versus less than ten for sequential polypeptides) and the sequence of their block repeats can be almost infinitely complex. Table A depicts examples of genetically engineered blocks. Table A and a further description of genetically engineered blocks may be found in Franco A. Ferrari and Joseph Cappello, Biosynthesis of Protein Polymers, in: Protein-Based Materials, (eds., Kevin McGrath and David Kaplan), Chapter 2, pp. 37-60, Birkhauser, Boston (1997).

**Table A. Protein polymer sequences**

| Polymer Name | Monomer Amino Acid Sequence |
|---|---|
| SLP 3 | [(GAGAGS)₉ GAAGY)] |
| SLP 4 | (GAGAGS)ₙ |
| SLP F | [(GAGAGS)₉ GAA VTGRGDSPAS AAGY]ₙ |
| SLP L3.0 | [(GAGAGS)₉ GAA PGASIKVAVSAGPS AGY]ₙ |
| SLP L3.1 | [(GAGAGS)₉ GAA PGASIKVAVSGPS AGY]ₙ |
| SLP F9 | [(GAGAGS)₉ RYWLPRPVCFEK AAGY]ₙ |
| ELPI | [(VPGVG)₄]ₙ |
| SELP 0 | [(GVGVP)₈ (GAGAGS)₂]ₙ |
| SELP 1 | [GAA (VPGVG)₄ VAAGY (GAGAGS)₉]ₙ |
| SELP 2 | [(GAGAGS)₆ GAAGY (GAGAGS)₅ (GVGVP)₈]ₙ |
| SELP 3 | [(GVGVP)₈ (GAGAGS)₈]ₙ |
| SELP 4 | [(GVGVP)₁₂ (GAGAGS)₈]ₙ |
| SELP 5 | [(GVGVP)₁₆ (GAGAGS)₈]ₙ |
| SELP 6 | [(GVGVP)₃₂ (GAGAGS)₈]ₙ |
| SELP 7 | [(GVGVP)₈ (GAGAGS)₆]ₙ |
| SELP 8 | [(GVGVP)₈ (GAGAGS)₄]ₙ |
| KLP 1.2 | [(AKLKLAEAKLELAE)₄]ₙ |
| CLP 1 | [GAP(GPP)₄]ₙ |
| CLP 2 | {[GAP(GPP)₄]₂ GPAGPVGSP}ₙ |
| CLP-CB | {[GAP(GPP)₄]₂ (GLPGPKGDRGDAGPKGADGSPGPA) GPAGPVGSP}ₙ |
| CLP 3 | (GAPGAPGSQGAPGLQ)ₙ |

Repetitive amino acid sequences of selected protein polymers. SLP = silk like protein; SLPF = SLP containing the RGD sequence from fibronectin; SLPL 3/0 and SLPL 3/1 = SLP containing two difference sequences from laminin protein; ELP = elastin like protein; SELP = silk elastin like protein; CLP = collagen like protein; CLP-CB =CLP containing a cell binding domain from human collagen; KLP = keratin like protein

The nature of the elastinlike blocks, and their length and position within the monomers influences the water solubility of the SELP polymers. For example, decreasing the length and/or content of the silklike block domains, while maintaining the length of the elastinlike block domains, increases the water solubility of the polymers. For a more detailed discussion of the production of SLP's, ELP's, CLP's, SLPF's and SELP's as well as their properties and characteristics see, for example, in J. Cappello et al., Biotechnol. Prog., 6, 198 (1990), the full disclosure of which is incorporated by reference herein. One preferred SELP, SELP7, has an elastin:silk ratio of 1.33, and has 45% silklike protein material and is believed to have weight average molecular weight of 80,338.

The amount of the biocompatible protein component utilized in the coatable composition will be dependent upon the amount of coatable composition desired in relation to the other components of the device and the particular biocompatible protein component chosen for use in the coatable composition. Furthermore, the amount of coatable composition utilized in the coating of the film will be determinative of the size of the film, and thus, the size of the cohesive body and the resulting protein matrix device. That is, inasmuch as the amounts of the remaining components are dependent upon the amount of biocompatible protein component utilized, the amount of biocompatible protein component may be chosen based upon the aforementioned parameters.

Any biocompatible solvent may be utilized in the method and corresponding protein matrix device of the present invention. By using a biocompatible solvent, the risk of adverse tissue reactions to residual solvent remaining in the device after manufacture is minimized. Additionally, the use of a biocompatible solvent reduces the potential structural and/or pharmacological degradation of the optional pharmacologically active agents that some such pharmacologically active agents undergo when exposed to organic solvents. Suitable biocompatible solvents for use in the method of the present invention include, but are not limited to, water; dimethyl sulfoxide (DMSO); biocompatible alcohols, such as methanol and ethanol; various acids, such as formic acid; oils, such as olive oil, peanut oil and the like; ethylene glycol, glycols; and combinations of these and the like. Preferably, the biocompatible solvent comprises water. The amount ofbiocompatible solvent utilized in the coatable composition will preferably be that amount sufficient to result in the composition being fluid and flowable enough to be coatable. Generally, the amount of biocompatible solvent suitable for use in the method of the present invention will range from about 50% to about 500%, preferably from about 100% to about 300% by weight, based upon the weight of the biodegradable polymeric material.

In addition to the biocompatible protein material(s) and the biocompatible solvent(s), the protein matrix devices of the present invention may optionally comprise one or more pharmacologically active agents. As used herein, "pharmacologically active agent" generally refers to a pharmacologically active agent having a direct or indirect beneficial therapeutic effect upon introduction into a host. Pharmacologically active agents further includes neutraceuticals. The phrase "pharmacologically active agent" is also meant to indicate prodrug forms thereof. A "prodrug form" of a pharmacologically active agent means a structurally related compound or derivative of the pharmacologically active agent which, when administered to a host is converted into the desired pharmacologically active agent. A prodrug form may have little or none of the desired pharmacological activity exhibited by the pharmacologically active agent to which it is converted. Representative examples of pharmacologically active agents that may be suitable for use in the protein matrix device of the present invention include, but are not limited to, (grouped by therapeutic class):
Antidiarrhoeals such as diphenoxylate, loperamide and hyoscyamine;
Antihypertensives such as hydralazine, minoxidil, captopril, enalapril, clonidine, prazosin, debrisoquine, diazoxide, guanethidule, methyldopa, reserpine, trimethaphan;
Calcium channel blockers such as diltiazem, felodipine, amodipine, nitrendipine, nifedipine and verapamil;
Antiarrhyrthmics such as amiodarone, flecainide, disopyramide, procainamide, mexiletene and quinidine;
Antiangina agents such as glyceryl trinitrate, erythrityl tetranitrate, pentaerythritol tetranitrate, mannitol hexanitrate, perhexilene, isosorbide dinitrate and nicorandil;
Beta-adrenergic blocking agents such as alprenolol, atenolol, bupranolol, carteolol, labetalol, metoprolol, nadolol, nadoxolol, oxprenolol, pindolol, propranolol, sotalol, timolol and timolol maleate;
Cardiotonic glycosides such as digoxin and other cardiac glycosides and theophylline derivatives;
Adrenergic stimulants such as adrenaline, ephedrine, fenoterol, isoprenaline, orciprenaline, rimeterol, salbutamol, salmeterol, terbutaline, dobutamine, phenylephrine, phenylpropanolamine, pseudoephedrine and dopamine;
Vasodilators such as cyclandelate, isoxsuprine, papaverine, dipyrimadole, isosorbide dinitrate, phentolamine, nicotinyl alcohol, co-dergocrine, nicotinic acid, glycerl trinitrate, pentaerythritol tetranitrate and xanthinol;
Antimigraine preparations such as ergotanmine, dihydroergotamine, methysergide, pizotifen and sumatriptan;
Anticoagulants and thrombolytic agents such as warfarin, dicoumarol, low molecular weight hepafins such as enoxaparin, streptokinase and its active derivatives;
Hemostatic agents such as aprotinin, tranexamic acid and protamine;
Analgesics and antipyretics including the opioid analgesics such as buprenorphine, dextromoramide, dextropropoxyphene, fentanyl, alfentanil, sufentanil, hydromorphone, methadone, morphine, oxycodone, papaveretum, pentazocine, pethidine, phenopefidine, codeine dihydrocodeine; acetylsalicylic acid (aspirin), paracetamol, and phenazone;
Neurotoxins such as capsaicin;
Hypnotics and sedatives such as the barbiturates amylobarbitone, butobarbitone and pentobarbitone and other hypnotics and sedatives such as chloral hydrate, chlormethiazole, hydroxyzine and meprobamate;
Antianxiety agents such as the benzodiazepines alprazolam, bromazepam, chlordiazepoxide, clobazam, chlorazepate, diazepam, flunitrazepam, flurazepam, lorazepam, nitrazepam, oxazepam, temazepam and triazolam;
Neuroleptic and antipsychotic drugs such as the phenothiazines, chlorpromazine, flupbenazine, pericyazine, perphenazine, promazine, thiopropazate, thioridazine, trifluoperazine; and butyrophenone, droperidol and haloperidol; and other antipsychotic drugs such as pimozide, thiothixene and lithium;
Antidepressants such as the tricyclic antidepressants amitryptyline, clomipramine, desipramine, dothiepin, doxepin, imipramine, nortriptyline, opipramol, protriptyline and trimipramine and the tetracyclic antidepressants such as mianserin and the monoamine oxidase inhibitors such as isocarboxazid, phenelizine, tranylcypromine and moclobemide and selective serotonin re-uptake inhibitors such as fluoxetine, paroxetine, citalopram, fluvoxamine and sertraline;
CNS stimulants such as caffeine and 3-(2-aminobutyl) indole;
Anti-alzheimer's agents such as tacrine;
Anti-Parkinson's agents such as amantadine, benserazide, carbidopa, levodopa, benztropine, bipefiden, benzhexol, procyclidine and dopamine-2 agonists such as S (-)-2 -(N-propyl -N-2 -thi enyl ethyl amino)-5 -hydroxytetralin (N-0923)-;
Anticonvulsants such as phenytoin, valproic acid, primidone, phenobarbitone, methylphenobarbitone and carbamazepine, ethosuximide, methsuximide, phensuximide, sulthiame and clonazepam;
Antiemetics and antinauseants such as the phenothiazines prochloperazine, thiethylperazine and 5HT-3 receptor antagonists such as ondansetron and granisetron, as well as dimenhydrinate, diphenhydramine, metoclopramide, domperidone, hyoscine, hyoscine hydrobromide, hyoscine hydrochloride, clebopride and brompride;
Non-steroidal anti-inflammatory agents including their racemic mixtures or individual enantiomers where applicable, preferably which can be formulated in combination with dermal penetration enhancers, such as ibuprofen, flurbiprofen, ketoprofen, aclofenac, diclofenac, aloxiprin, aproxen, aspirin, diflunisal, fenoprofen, indomethacin, mefenamic acid, naproxen, phenylbutazone, piroxicam, salicylamide, salicylic acid, sulindac, desoxysulindac, tenoxicam, tramadol, ketoralac, flufenisal, salsalate, triethanolamine salicylate, atninopyrine, antipyrine, oxyphenbutazone, apazone, cintazone, flufenamic acid, clonixerl, clonixin, meclofenamic acid, flunixin, colchicine, demecolcine, allopurinol, oxypurinol, benzydamine hydrochloride, dimefadane, indoxole, intrazole, mimbane hydrochloride, paranylene hydrochloride, tetrydamine, benzindopyrine hydrochloride, fluprofen, ibufenac, naproxol, fenbufen, cinchophen, diflumidone sodium, fenamole, flutiazin, metazamide, letimide hydrochloride, nexeridine hydrochloride, octazamide, molinazole, neocinchophen, nimazole, proxazole citrate, tesicam, tesimide, tolmetin, and triflumidate;
Antirheumatoid agents such as penicillamine, aurothioglucose, sodium aurothiomalate, methotrexate and auranofin;
Muscle relaxants such as baclofen, diazepam, cyclobenzaprine hydrochloride, dantrolene;
methocarbamol, orphenadrine and quinine;
Agents used in gout and hyperuricaemia such as allopurinol, colchicines, probenecid and sulphinpyrazone;
Oestrogens such as oestradiol, oestriol, oestrone, ethinyloestradiol, mestranol, stilboestrol, dienoestrol, epioestriol, estropipate and zeranol;
Progesterone and other progestagens such as allyloestrenol, dydrgesterone, lynoestrenol;
norgestrel, norethyndrel, norethisterone, norethisterone acetate, gestodene, levonorgestrel;
medroxyprogesterone and megestrol;
Antiandrogens such as cyproterone acetate and danazol;
Antioestrogens such as tamoxifen and epitiostanol and the aromatase inhibitors, exemestane and 4-hydroxy-androstenedione and its derivatives;
Androgens and anabolic agents such as testosterone, methyltestosterone, clostebol acetate, drostanolone, furazabol, nandrolone oxandrolone, stanozolol, trenbolone acetate, dihydro-testosterone;
17-(a-methyl-19-noriestosterone and fluoxymesterone;
5-alpha reductase inhibitors such as finastride, turosteride, LY- 191704 and MK-306-1;
Corticosteroids such as betamethasone, betamethasone valerate, cortisone, dexamethasone, dexamethasone 21-phosphate, fludrocortisone, flumethasone, fluocinonide, fluocinonide desonide, fluocinolone, fluocinolone acetonide, fluocortolone, halcinonide, halopredone, hydrocortisone, hydrocortisone 17-valerate, hydrocortisone 17-butyrate, hydrocortisone 21-acetate, methylprednisolone, prednisolone, prednisolone 21 -phosphate, prednisone, triamcinolone, triamcinolone acetonide;
Glycosylated proteins, proteoglycans, glycosaminoglycans such as chondroitin sulfate; chitin, acetyl-glucosamine, hyaluronic acid;
Complex carbohydrates such as glucans;
Further examples of steroidal anti-inflammatory agents such as cortodoxone, fludroracetonide, fludrocortisone, difluorsone diacetate, flurandrenolone acetonide, medrysone, amcinafel, amcinafide, betamethasone and its other esters, chloroprednisone, cloreortelone, descinolone, desonide, dichlofisone, difluprednate, flucloronide, flumethasone, flunisolide, flucortolone, fluoromethalone, fluperolone, fluprednisolone, meprednisone, methylmeprednisolone, paramethasone, cortisone acetate, hydrocortisone cyclopentylpropionate, cortodoxone, flucetonide, fludrocortisone acetate, amcinafal, amcinafide, betamethasone, betamethasone benzoate, chloroprednisone acetate, clocortolone acetate, descinolone acetonide, desoximetasone, dichlorisone acetate, difluprednate, flucloronide, flumethasone pivalate, flunisolide acetate, fluperolone acetate, fluprednisolone valerate, paramethasone acetate, prednisolamate, prednival, triamcinolone hexacetonide, cortivazol, formocortal and nivazoll;
Pituitary hormones and their active derivatives or analogs such as corticotrophin, thyrotropin, follicle stimulating hormone (FSH), luteinising hormone (LH) and gonadotrophin releasing hormone (GnRH);
Hypoglycemic agents such as insulin, chlorpropamide, glibenclamide, gliclazide, glipizide, tolazamide, tolbutamide and metformin;
Thyroid hormones such as calcitonin, thyroxine and liothyronine and antithyroid agents such as carbimazole and propylthiouracil;
Other miscellaneous hormone agents such as octreotide;
Pituitary inhibitors such as bromocriptine;
Ovulation inducers such as clomiphene;
Diuretics such as the thiazides, related diuretics and loop diuretics, bendrofluazide, chlorothiazide, chlorthalidone, dopamine, cyclopenthiazide, hydrochlorothiazide, indapamide, mefruside, methycholthiazide, metolazone, quinethazone, bumetanide, ethacrynic acid and frusemide and potasium sparing diuretics, spironolactone, amiloride and triamterene;
Antidiuretics such as desmopressin, lypressin and vasopressin including their active derivatives or analogs;
Obstetric drugs including agents acting on the uterus such as ergometfine, oxytocin and gemeprost;
Prostaglandins such as alprostadil (PGEI), prostacyclin (PG12), dinoprost (prostaglandin F2-alpha) and misoprostol;
Antimicrobials including the cephalospofins such as cephalexin, cefoxytin and cephalothin;
Penicillins such as amoxycillin, amoxycillin with clavulanic acid, ampicillin, bacampicillin, benzathine penicillin, benzylpenicillin, carbencillin, cloxacillin, methicillin, phenethicillin, phenoxymethylpenicillin, flucloxacillin, meziocillin, piperacillin, ticarcillin and azlocillin;
Tetracyclines such as minocycline, chlortetracycline, tetracycline, demeclocycline, doxycycline, methacycline and oxytetracycline and other tetracycline-type antibiotics;
Amnioglycoides such as amikacin, gentamicin, kanamycin, neomycin, netilmicin and tobramycin;
Antifungals such as amorolfine, isoconazole, clotrimazole, econazole, miconazole, nystatin, terbinafine, bifonazole, amphotericin, griseofulvin, ketoconazole, fluconazole and flucytosine, salicylic acid, fezatione, ticlatone, tolnaftate, triacetin, zinc, pyrithione and sodium pyfithione;
Quinolones such as nalidixic acid, cinoxacin, ciprofloxacin, enoxacin and norfloxacin;
Sulphonamides such as phthalysulphthiazole, sulfadoxine, sulphadiazine, sulphametluzole and sulphamethoxazole;
Sulphones such as dapsone;
Other miscellaneous antibiotics such as chloramphenicol, clindamycin, erythromycin, erythromycin ethyl carbonate, erythromycin estolate, erythromycin glucepate, erythromycin ethylsuccinate, erythromycin lactobionate, roxithromycin, lincomycin, natamycin, nitrofurantoin, spectinomycin, vancomycin, aztreonam, colistin IV, metronidazole, tinidazole, fusidic acid, trimethoprim, and 2-thiopyridine N-oxide; halogen compounds, particularly iodine and iodine compounds such as iodine-PVP complex and diiodohydroxyquin, hexachlorophene; chlorhexidine; chloroan-tine compounds; and benzoylperoxide;
Antituberculosis drugs such as ethambutol, isoniazid, pyrazinamide, rifampicin and clofazimine;
Antimalarials such as primaquine, pyrimethamine, chloroquine, hydroxychloroquine, quinine, mefloquine and halofantrine;
Antiviral agents such as acyclovir and acyclovir prodrugs, famcyclovir, zidovudine, didanosine, stavudine, lamivudine, zalcitabine, saquinavir, indinavir, ritonavir, n-docosanol, tromantadine and idoxuridine;
Anthelinintics such as mebendazole, thiabendazole, niclosamide, praziquantel, pyrantel embonate and diethylcarbamazine;
Cytotoxic agents such as plicamycin, cyclophosphamide, dacarbazine, fluorouracil and its prodrugs (described, for example, in International Journal-of Pharmaceutics, 111 223-233 (1994)), methotrexate, procarbazine, 6-mercaptopurine and mucophenolic acid;
Anorectic and weight reducing agents including dexfenfluramine, fenfluramine, diethylpropion, mazindol and phentermine;
Agents used in hypercalcaemia such as calcitriol, dihydrotachysterol and their active derivatives or analogs;
Antitussives such as ethylmorphine, dextromethorphan and pholcodine;
Expectorants such as carbolcysteine, bromhexine, emetine, quanifesin, ipecacuanha and saponins;
Decongestants such as phenylephrine, phenylpropanolamine and pseudoephedrine;
Bronchospasm relaxants such as ephedrine, fenoterol, orciprenaline, rimiterol, salbutamol, sodium cromoglycate, cromoglycic acid and its prodrugs (described, for example, in International Journal of Pharmaceutics 7, 63-75 (1980*)),* terbutaline, ipratropium bromide, salmeterol and theophylline and theophylline derivatives;
Antihistamines such as meclozine, cyclizine, chlorcyclizine, hydroxyzine, brompheniramine, chlorpheniramiine, clemastine, cyproheptadine, dexchlorpheniramine, diphenhydramine, diphenylamine, doxylatnine, mebhydrolin, pheniramine, tripolidine, azatadine, diphenylpyraline, methdilazine, terfenadine, astemizole, loratidine and cetirizine;
Local anaesthetics such as bupivacaine, amethocaine, lignocaine, lidocaine, cinchocaine, dibucaine, mepivacaine, prilocaine, etidocaine, veratridine (specific c-fiber blocker) and procaine;
Stratum corneum lipids, such as ceramides, cholesterol and free fatty acids, for improved skin barrier repair [Man, et al. J.Invest. Dermatol., 106(5), 1096, (1996)];
Neuromuscular blocking agents such as suxamethonium, alcuronium, pancuronium, atracurium, gallamine, tubocurarine and vecuronium;
Smoking cessation agents such as nicotine, bupropion and ibogaine;
Insecticides and other pesticides which are suitable for local application;
Dermatological agents, such as vitamins A, C, B1, B2, B6, B12., and E, vitamin E acetate and vitamin E sorbate;
Allergens for desensitisation such as house, dust or mite allergens;
Nutritional agents and neutraceuticals, such as vitamins, essential amino acids and fats;
acromolecular pharmacologically active agents such as proteins, enzymes, peptides, polysaccharides (such as cellulose, amylose, dextran, chitin), nucleic acids, cells, tissues, and the like; and
Keratolytics such as the alpha-hydroxy acids, glycolic acid and salicylic acid.

The protein matrix devices as disclosed herein may also be utilized for DNA delivery, either naked DNA, plasma DNA or any size DNA delivery. Also, the protein matrix may be utilized for delivery of RNA types of senses, or oligonucleotides that may be man-made portions of DNA or RNA. The protein matrix could also be utilized for delivery of compounds, as explained anywhere herein, in ovum or in embryos, as the site for implantation of the protein matrix.

The DNA, RNA or oligonucleotide may be incorporated into the protein matrix utilizing the same process of making the protein matrix device as described above. The only difference would be that the pharmacological active agents utilized would be the DNA, RNA, oligonucleotides and other such materials. In one example, a cohesive body may be produced by making a composition containing one or more biocompatible proteins, one or more biocompatible solvents and an antisense type material. In general the complementary strand of a coding sequence of DNA is the cDNA and the complementary strand of mRNA is the antisense RNA. In various embodiments of the present invention, antisense material delivered by a protein matrix device of the present invention binds with mRNA, thereby preventing it from making the protein.

Two of the advantages of including DNA, RNA or oligonucleotides in a protein matrix device is that such a device includes the benefits of local drug delivery to target cells and to have a controlled time release component so that there is an extended delivery period. An additional advantage to delivery of DNA, RNA or oligonucleotides components is that the DNA, RNA or oligonucleotides components can be released in a systematic and controlled manner over a long period of time. For example, when the antisense components bind with RNA, the body tends to cleave the RNA thereby inhibiting protein production. The biological system responds by making more RNA to make proteins. The protein matrix device provides delivery of additional antisense components in a location for an extended period of time, thereby blocking the production of the undesired protein. Also the biocompatibility of the protein matrix material enhances the binding characteristics of the anitsense components to their proper binding sites. Since the protein matrix material can be fabricated or produced to resemble the host tissue, the host cells are able to better interact with the administered protein matrix device, thereby facilitating the binding of the complimentary antisense components delivered by the protein matrix with the DNA and RNA in the host cells.

Additionally, the use of a protein matrix device in an egg or womb could be very useful for a number of applications. For example, a vaccine may be delivered in ova and then released into the animal, such as mammals, birds or reptiles, even after it's born. Also, the introduction of pharmacologically active agents that could be put in the egg or womb, could be beneficial in that it could inhibit things like bacteria or viral infection of the egg or womb during incubation and promote the healthy development of a mature animal. For example, it would be possible for the protein matrix device to act as a drug delivery device for growth factors, neutraceuticals like vitamins or other agents that would help in the growth of the animal after it's hatched, or even during the stage when it is unhatched to facilitate the development of that animal. Another example would be the production of livestock, such as domestic animals like horses, cattle, pigs, sheep, dogs, cats, chickens or turkeys. If domestic animals would get a head start on growth, it may enhance their body weight, which would have a tremendous impact on the overall development of the specimen.

Finally, protein matrix devices may be produced in particulate forms. These forms comprise vaccine particles of all types, including protein particles containing antigen components that may be made small enough (2-10µm) to be absorbed by immunogenic cells for enhanced immune response via subcutaneous, intraparetaneal, intravenous, intramuscular, intrathecal, epidural, intraarticular or any other administration delivery means.

The protein matrix device in accordance with the present invention, as mentioned hereinabove, may comprise an amount of a neurotoxin as the pharmacologically active agent. Specifically, inasmuch as some cases of chronic pain are the result of permanent nerve damage, in some instances it may be desirable to locally deliver an amount of a neurotoxin to the injured nerve to destroy that portion of the nerve that is the cause of the persistent, chronic pain. One example of a neurotoxin suitable for use in the present invention is capsaicin. If a neurotoxin is to be incorporated into the protein matrix device of the present invention, it is preferred that it be incorporated in an amount ranging from about 0.001% to about 5%, more preferably, from about 0.05% to about 1% by weight, based upon the weight of the biocompatible protein component.

The protein matrix device of the present invention is particularly advantageous for the encapsulation/incorporation of macromolecular pharmacologically active agents such as proteins, enzymes, peptides, polysaccharides, nucleic acids, cells, tissues, and the like. Immobilization of macromolecular pharmacologically active agents into or onto a protein matrix device can be difficult due to the ease with which some of these macromolecular agents denature when exposed to organic solvents, some constituents present in bodily fluids or to temperatures appreciably higher than room temperature. However, since the method of the present invention, as well as the protein matrix device formed by the method utilizes biocompatible solvents such as water, DMSO or ethanol, and furthermore does not require heating, the risk of the denaturation of these types of materials is reduced. Furthermore, due to the size of these macromolecular pharmacologically active agents, these agents are encapsulated within the protein matrix upon implantation of protein matrix devices in accordance with the present invention, and thereby are protected from constituents of bodily fluids that would otherwise denature them. Thus, the protein matrix devices of the present invention allow these macromolecular agents may exert their therapeutic effects, while yet protecting them from denaturation or other structural degradation.

Examples of cells which can be utilized as the pharmacologically active agent in the protein matrix device of the present invention include primary cultures as well as established cell lines, including transformed cells. Examples of these include, but are not limited to pancreatic islet cells, human foreskin fibroblasts, Chinese hamster ovary cells, beta cell insulomas, lymphoblastic leukemia cells, mouse 3T3 fibroblasts, dopamine secreting ventral mesencephalon cells, neuroblastold cells, adrenal medulla cells, T-cells combinations of these, and the like. The growth of endothelial and smooth muscle cells on embodiments of the protein matrix devices of the present invention may also provide additional biocompatible benefits. As can be seen from this partial list, cells of all types, including dermal, neural, blood, organ, stem, muscle, glandular, reproductive and immune system cells, as well as cells of all species of origin, can be encapsulated successfully by this method.

Examples of proteins which can be incorporated into the protein matrix device of the present invention include, but are not limited to, hemoglobin, vasporessin, oxytocin, adrenocorticocotrophic hormone, epidermal growth factor, prolactin, luliberin or luteinising hormone releasing factor, human growth factor, and the like; enzymes such as adenosine deaminase, superoxide dismutase, xanthine oxidase, and the like; enzyme systems; blood clotting factors; clot inhibitors or clot dissolving agents such as streptokinase and tissue plasminogen activator; antigens for immunization; hormones; polysaccharides such as heparin; oligonucleotides; bacteria and other microbial microorganisms including viruses; monoclonal antibodies; vitamins; cofactors; retroviruses for gene therapy, combinations of these and the like.

An efficacious amount of the aforementioned pharmacologically active agent(s) can easily be determined by those of ordinary skill in the art taking into consideration such parameters as the particular pharmacologically active agent chosen, the size and weight of the patient, the desired therapeutic effect, the pharmacokinetics of the chosen pharmacologically active agent, and the like, as well as by reference to well known resources such as Physicians' Desk Reference®: PDR--52 ed (1998)--Medical Economics 1974. In consideration of these parameters, it has been found that a wide range exists in the amount of the pharmacologically active agent(s) capable of being incorporated into, and subsequently released from or alternatively allowed to exert the agent's therapeutic effects from within, the protein matrix device. More specifically, the amount of pharmacologically active agent that may be incorporated into and then either released from or active from within the protein matrix device may range from about 0.001% to about 200%, more preferably, from about 0.05% to about 100%, most preferably from about 0. 1% to 70%, based on the weight of the biocompatible protein material.

In addition to the biocompatible protein material(s), the biocompatible solvent(s) and pharmacologically active agent(s), the protein matrix devices of the present invention advantageously may themselves incorporate other drug delivery devices that would otherwise typically migrate away from the desired delivery site and/or are potentially undesirably reactive with surrounding bodily fluids or tissues. Such migration is undesirable in that the therapeutic effect of the pharmacological agents encapsulated therein may occur away from the desired site, thus eliminating the advantage of localized delivery. When a protein matrix device incorporating a migration-vulnerable and/or reactive drug delivery device (hereinafter referred to as a "two-stage protein matrix device") is subsequently implanted, the migration-vulnerable and/or reactive drug delivery device(s) is/are held in place and protected by the two-stage protein matrix device. More particularly, once implanted and/or administered, the pharmacologically active agent is released by the biodegradable material of the migration-vulnerable drug delivery devices as it degrades. Then the pharmacologically active agents diffuse through the protein matrix of the two-stage protein matrix device or is released with the degradation of the protein matrix device of the present invention.

Furthermore, the compressed cohesive body of the protein matrix device reduces, if not prevents, the potential for undesirable reaction with bodily fluids or tissues that may otherwise occur upon implantation of a reactive drug delivery device without the protective protein matrix encapsulation. Examples of such drug delivery devices subject to migration for the delivery site include, but are not limited to, vesicles, e.g., liposomes, lipospheres and microspheres. Vesicles are made up of microparticles or colloidal carriers composed of lipids, carbohydrates or synthetic polymer matrices and are commonly used in liquid drug delivery devices. Vesicles, for example, have been used to deliver anesthetics using formulations with polylactic acid, lecithin, iophendylate and phosphotidyl choline and cholesterol. For a discussion of the characteristics and efficiency of drug delivery from vesicles, see, e.g., Wakiyama et al., Chem., Pharm. Bull., 30, 3719 (1982*)* and Haynes et al., Anesthiol, 74, 105 (1991), the entire disclosures of which are incorporated by reference herein.

Liposomes, the most widely studied type of vesicle, can be formulated to include a wide variety of compositions and structures that are potentially non-toxic, biodegradable and non-immunogenic. Furthermore, studies are in progress to create liposomes that release more drug in response to changes in their environment, including the presence of enzymes or polycations or changes in pH. For a review of the properties and characteristics of liposomes see, e.g., Langer, Science, 249, 1527 (1990); and Langer, Ann. Biomed. Eng., 23, 101 (1995), the entire disclosures of which are incorporated by reference herein.

Lipospheres are an aqueous microdispersion of water insoluble, spherical microparticles (from about 0.2 to about 100 um in diameter), each consisting of a solid core of hydrophobic triglycerides and drug particles that are embedded with phospholipids on the surface. Lipospheres are disclosed in U.S. Patent No. 5,188,837, issued to Domb, the disclosure of which is incorporated herein by reference.

Microspheres typically comprise a biodegradable polymer matrix incorporating a drug. Microspheres can be formed by a wide variety of techniques known to those of skill in the art. Examples of microsphere forming techniques include, but are not limited to, (a) phase separation by emulsification and subsequent organic solvent evaporation (including complex emulsion methods such as oil in water emulsions, water in oil emulsions and water-oil-water emulsions); (b) coacervation-phase separation; (c) melt dispersion; (d) interfacial deposition; (e) *in situ* polymerization; (f) spray drying and spray congealing; (g) air suspension coating; and (h) pan and spray coating. These methods, as well as properties and characteristics of microspheres are disclosed in, e.g., U.S. Pat. No. 4,652,441; U.S. Pat. No. 5,100,669; U.S. Pat. No. 4,526,938; WO 93/24150; EPA 0258780 A2- U.S. Pat. No. 4,438,253; and U.S. Patent 5,330,768, the entire disclosures of which are incorporated by reference herein.

Inasmuch as the migration-vulnerable and/or reactive drug delivery devices will desirably further encapsulate a pharmacologically active agent, the amount of these devices to be utilized in the two-stage protein matrix device may be determined by the dosage of the pharmacologically active agent, as determined and described hereinabove. Inasmuch as such migration-vulnerable and/or reactive drug delivery devices represent solid matter that may change the ability of the coatable composition to be coated, the amount of such devices to be included in a two-stage drug delivery device desirably ranges about 10,000 to about 1 billion, more preferably ranges from about 1 million to about 500 million, and most preferably ranges from about 200 million to about 400 million.

Additionally, the protein matrix devices formed according to the method of the present invention may optionally comprise one or more additives. Such additives may be utilized, for example, to facilitate the processing of the protein matrix devices, to stabilize the pharmacologically active agents, to facilitate the activity of the pharmacologically active agents, or to alter the release characteristics of the protein matrix device. For example, when the pharmacologically active agent is to be an enzyme, such as xanthine oxidase or superoxide dismutase, the protein matrix device may further comprise an amount of an enzyme substrate, such as xanthine, to facilitate the action of the enzyme.

Additionally, hydrophobic substances such as lipids can be incorporated into the protein matrix device to extend the duration of drug release, while hydrophilic, polar additives, such as salts and amino acids, can be added to facilitate, i.e., shorten the duration of, drug release. Exemplary hydrophobic substances include lipids, e.g., tristearin, ethyl stearate, phosphotidycholine, polyethylene glycol (PEG); fatty acids, e.g., sebacic acid erucic acid; combinations of these and the like. A particularly preferred hydrophobic additive useful to extend the release of the pharmacologically active agents comprises a combination of a dimer of erucic acid and sebacic acid, wherein the ratio of the dimer of erucic acid to sebacic acid is 1:4. Exemplary hydrophilic additives useful to shorten the release duration of the pharmacologically active agent include but are not limited to, salts, such as sodium chloride; and amino acids, such as glutamine and glycine. If additives are to be incorporated into the coatable composition, they will preferably be included in an amount so that the desired result of the additive is exhibited. Generally, the amount of additives may vary between from about 0% to about 300%, preferably from about 100% to 200% by weight, based upon the weight of the biocompatible protein material.

Manufacturing protein matrix devices with the method of the present invention imparts many advantageous qualities to the resulting protein matrix devices. First of all, by compressing the cohesive body in such a manner, the resulting protein matrix device is substantially cohesive and durable, i.e., with a solvent content of from about 10% to about 60%, preferably of from about 30% to about 50%. Thus, administration of the protein matrix device is made easy, inasmuch as it may be easily handled to be injected or implanted. Furthermore, once implanted, the biocompatible protein material may absorb water and swell, thereby assisting the protein matrix device to stay substantially in the location where it was implanted or injected. Additionally, since the protein material may be biodegradable and the pharmacologically active agent is distributed substantially homogeneously therein, the release kinetics of the pharmacologically active agent are optimized. Indeed, the components and the amounts thereof to be utilized in the protein matrix device may be selected so as to optimize the rate of delivery of the pharmacologically active agent depending upon the desired therapeutic effect and pharmacokinetics of the chosen pharmacologically active agent.

Finally, since biocompatible solvents are used in the manufacture of the protein matrix devices, the potential for adverse tissue reactions to chemical solvents are reduced, if not substantially precluded. For all of these reasons; protein matrix devices in accordance with the present invention may advantageously be used to effect a local therapeutic result in a patient in need of such treatment. More specifically, the protein matrix devices of the present invention may be injected, implanted, or administered via oral, as well as nasal, subcutaneous, or any other parenteral mode of delivery. The protein matrix device may be delivered to a site within a patient to illicit a therapeutic effect either locally or systemically. Depending on the desired therapeutic effect, the protein matrix devices that include pharmacologically active agents and/or conductive materials may be used to regenerate tissue or bone, repair tissue or bone, replace tissueor bone, and deliver local and systemic therapeutic effects such as analgesia or anesthesia, or alternatively, may be used to treat specific conditions, such as coronary artery disease, heart valve failure, cornea trauma, skin, tissue and bone wounds and other tissue or bone specific conditions. Protein matrix devices that include pharmacologically active agents and/or conductive materials may be utilized in instances where long term, sustained, controlled release of pharmacologically active agents or applied activation of electrical energy, heat and/or electrical current is desirable. Examples wherein such treatments are utilize include but are not limited to the treatment of surgical and post-operative pain, cancer pain, other conditions requiring chronic pain management, tissue or bone repair and tissue or bone regeneration..

Furthermore, the protein matrix devices of the present invention may incorporate multiple pharmacologically active agents, one or more of which may be agents that are effective to suppress an immune and/or inflammatory response. In this regard, the protein matrix devices will deter, or substantially prevent the encapsulation that typically occurs when a foreign body is introduced into a host. Such encapsulation could potentially have the undesirable effect of limiting the efficacy of the protein matrix device.

Additionally, one or more polymeric materials may be included in the coatable composition to add or enhance the features of the protein matrix device. For example, one or more polymeric materials that degrades slowly may be incorporated into an embodiment of the protein matrix device that degrades in order to provide controllable release of a pharmacologically active agent that is also incorporated into the protein matrix device. That is, while a protein matrix device that includes a relatively fast-degrading protein material without a particular polymeric material will readily degrade thereby releasing drug relatively quickly upon insertion or implantation, a protein matrix device that includes a particular polymeric material, such as polyanhydride, will degrade slowly, as well as release the pharmacologically active agent(s) over a longer period of time. Examples of biodegradable and/or biocompatible polymeric materials suitable for use in the protein matrix device of the present invention include, but are not limited to epoxies, polyesters, acrylics, nylons, silicones, polyanhydride, polyurethane, polycarbonate, poly(tetrafluoroethylene) (PTFE), polycaprolactone, polyethylene oxide, polyethylene glycol, poly(vinyl chloride), polylactic acid, polyglycolic acid, polypropylene oxide, poly(akylene)glycol, polyoxyethylene, sebacic acid, polyvinyl alcohol (PVA), 2-hydroxyethyl methacrylate (HEMA), polymethyl methacrylate, 1,3-bis(carboxyphenoxy)propane, lipids, phosphatidylcholine, triglycerides, polyhydroxybutyrate (PHB), polyhydroxyvalerate (PHV), poly(ethylene oxide) (PEO), poly ortho esters, poly (amino acids), polycynoacrylates, polyphophazenes, polysulfone, polyamine, poly (amido amines), fibrin, graphite, flexible fluoropolymer, isobutyl-based, isopropyl styrene, vinyl pyrrolidone, cellulose acetate dibutyrate, silicone rubber, copolymers of these, and the like. Other materials that may be incorporated into the matrix that are not considered polymers, but provide enhanced features include, but are not limited to, ceramics, bioceramics, glasses bioglasses, glass-ceramics, resin cement, resin fill; more specifically, glass ionomer, hydroxyapatite, calcium sulfate, Al₂O₃, tricalcium phosphate, calcium phosphate salts, alginate and carbon.

As previously mentioned the protein matrix devices may include one or more conductive materials. Generally the conductive materials may be utilized to carry a current through the protein matrix devices thereby releasing polar pharmacological agents, providing heat, cold, a magnetic field and/or electrical treatment to an injured or diseased portions of the body, or to carry a current to a medical device imbedded within the protein matrix. The conductive materials may be in the form of particles, fibers, wires, meshes, screens, antennae or any other suitable form for transmitting an electrical current. Conductive materials include but are not limited to gold, silver, platinum, tungsten, stainless steel, nitinol, copper, niobium, titanium, ceramics or other like materials. Additional other materials that may be incorporated into the matrix included alloys such as, cobalt-based, galvanic- based, stainless steel- based, titanium- based, zirconium oxide, zirconia, aluminum- based, vanadium- based, molybdenum- based, nickel- based, iron- based, or zinc- based (zinc phosphate, zinc polycarboxylate).

Embodiments of the protein matrix device may also be crosslinked by reacting the components of the protein matrix with a suitable and biocompatible crosslinking agent. Crosslinking agents includes, but are not limited to glutaraldehyde, p-Azidobenzolyl Hydazide, N-5-Azido-2-nitrobenzoyloxysuccinimide, 4-[p-Azidosalicylamido]butylamine, any other suitable crosslinking agent and any combination thereof. A description and list of various crosslinking agents and a disclosure of methods of performing crosslinking steps with such agents may be found in the Pierce Endogen 2001-2002 Catalog which is hereby incorporated by reference.

Furthermore, it is noted that embodiments of the protein matrix device of the present invention may include crosslinking reagents that may be initiated and thereby perform the crosslinking process by UV light activation or other radiation source, such as ultrasound or gamma ray or any other activation means. Finally, the protein matrix devices may be crosslinked utilizing a polyfunctional aldehyde. For example, 2,2'-trimethylenebis-1,3-dioxolane may be used as a blocked polyfunctional aldehyde. Such a crosslinking technique utilizing a polyfunctional aldehyde is disclosed in U.S. Patent 6,177,514 and is incorporated by reference herein.

The protein matrix may be crosslinked by utilizing methods generally known in the art. For example, a protein matrix may be partially or entirely crosslinked by exposing, contacting and/or incubating the protein matrix device with a gaseous crosslinking reagent, liquid crosslinking reagent, light or combination thereof. In one embodiment of the present invention a tube may be crosslinked on the outside surface: by exposing the outside surface to a crosslinking reagent, such as glutaraldehyde. Such a matrix has the advantages of including an outer exterior that is very pliable and possesses greater mechanical characteristics, but includes an interior surface that retains higher biofunctional features. For example, cell growth may be controlled on portions of the protein matrix by exposing such areas to crosslinking reagents while still having portions of the same protein matrix that are not crosslinked, and thereby producing biofunctional selective features for the entire protein matrix device. For example crosslinking portions of the protein matrix device may be used to change, modify and/or inhibit cell attachment. It is also noted that the pharmacologically active agent may also be crosslinked, bonded and/or chemically and/or physically linked to protein matrix either partially or in totality such that the surface of the protein matrix and/or the interior of the protein matrix is linked to the protein matrix material. For example, glutaraldehyde may cross-link heparin to a single surface of a protein matrix device.

Embodiments of the present invention may include the addition of reagents to properly pH the resulting protein matrix device and thereby enhance the biocompatible characteristics of the device with the host tissue of which it is to be administered. When preparing the protein matrix device, the pH steps of the biocompatable material and biocompatable solvent occur prior to the partial drying preparation of the cohesive body. The pH steps can be started with the addition ofbiocompatable solvent to the protein material or to the mixture of protein material and optional biocompatible materials, or the pH steps can be started after mixing the material(s) and solvent(s) together before the cohesive body is formed. The pH steps can include the addition of drops of 0.05N to 4.0N acid or base to the solvent wetted material until the desired pH is reached as indicated by a pH meter, pH paper or any pH indicator. More preferably, the addition of drops of 0.1N-0.5 N acid or base are used. Although any acid or base may be used, the preferable acids and bases are HCl and NaOH, respectively. If known amounts of biocompatable protein material is used it may be possible to add acid or base to adjust the pH when the biocompatable protein material is first wetted, thereby allowing wetting and pH adjustments to occur in one step.

The patient to which the protein matrix device is administered may be any patient in need of a therapeutic treatment. Preferably, the patient is a mammal, reptiles and birds. More preferably, the patient is a human. Furthermore, the protein matrix device can be implanted in any location to which it is desired to effect a local therapeutic response. For example, the protein matrix device may be administered, applied, sutured, clipped, stapled, injected and/or implanted vaginally, in ova, in utero, in uteral, subcutaneously, near heart valves, in periodontal pockets, in the eye, in the intracranial space, next to an injured nerve, next to the spinal cord, etc. The present invention will now be further described with reference to the following non-limiting examples and the following materials and methods were employed. It is noted that any additional features presented in other embodiments described herein may be incorporated into the various embodiments being described.

### CURRENT RELEASED DRUG DELIVERY DEVICES

As previously suggested, various embodiments of the protein matrix device of the present invention may be utilized as current released drug delivery devices wherein the pharmacological agent(s) are released by an electrical current or a magnetic field. A drug delivery device produced and administered as previously disclosed or suggested includes the biocompatible features of the components of the protein matrix devices described above and thereby reduces or prevents the undesirable effects of toxicity and adverse tissue reactions that may be found in many other types of drug delivery devices. Furthermore, the controlled triggered release characteristics of this type of drug delivery device provides for a higher amount of pharmacologically active agent(s) that may be incorporated into the matrix. The controlled release of such a drug delivery device is partially attributed to the homogenous distribution of the pharmacologically active agent(s) throughout the drug delivery device and the triggering mechanism established by the passing of electricity through the device. This homogenous distribution provides for a more systematic, sustainable and consistent release of the pharmacologically active agent(s) by gradual degradation of the matrix or diffusion of the pharmacologically active agent(s) out of the matrix. As a result of the homogenous distribution and the current triggered release, the release characteristics of the pharmacologically active agent(s) from the protein matrix material and/or device are enhanced.

The current released drug delivery device may be prepared as previously described wherein the conductive material is either added to the coatable composition before coating the film or added to the cohesive body before compression. Generally, if the conductive material is added with the biocompatible protein, biocompatible solvent and pharmacologically active agent it is normally in a fibrous or particulate form. The fibrous or particulate form of conductive material allows the material to be homogenously distributed throughout the matrix with the other .components.thereby provided a uniform distribution throughout the entire protein matrix device. Alternatively, the conductive material may be added to the cohesive body before compression. When added to the cohesive body before compression the conductive material is generally in the form of wires or strands.

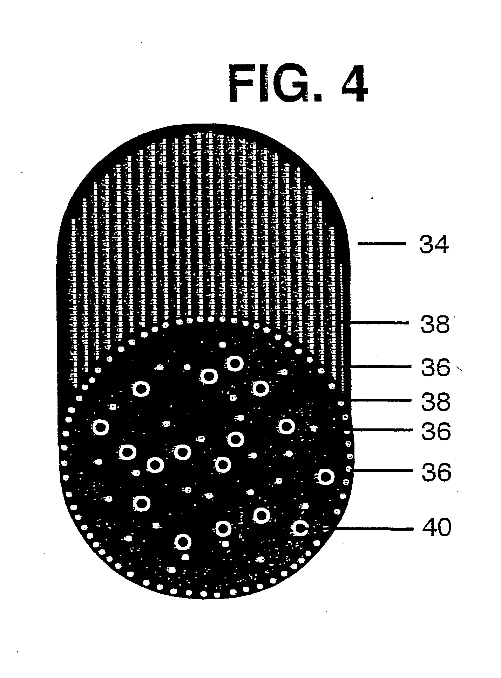

Figure 4 depicts an embodiment of the present invention wherein the drug delivery device 34 includes a series of conductive wires 36 positioned within the protein matrix material 38. The conductive wires 36 act as means for the transmission of an electrical current through the drug delivery device 34. As previously indicated the drug delivery device includes a homogenously distributed pharmacologically active agent 40 wherein release from the protein matrix material 38 is activated or enhanced by the flow of current through the drug delivery device 34.

Electrical current can affect drug diffusional kinetics so that drug release is increased by heat and slowed by cold. In various embodiments of the present invention heat can be generated by the passage of electrical current through the drug delivery device via the conductive material. Additionally, electrical polarization and increased electrostatic attraction within the matrix can decrease drug diffusion of polar chemicals, such as pharmacologically active agents like dexamethasone. Therefore, diffusion of such polar chemicals can be increased by passing an electrical current through the matrix.

Drugs that are electrostatically charged or associated/bonded to chemicals that are electrostatically charged will migrate with an electrical current. For example dexamethasone and drug associated chemical salts and or ions e.g. sodium-based, potassium-based, chlorine-based, magnesium-based compounds and other like compounds, will enhance release via electrical current activation. In this way a conductive material assembly can be incorporated into a protein matrix assembly that can facilitate drug release.

Also, electricity can set up a magnetic and/or electrical field to align polar chemicals thereby providing orientation to position chemicals within the matrix. This orientation can either increase attraction to the matrix to slow release of chemicals, such as pharmacologically active agents, and/or facilitate movement of chemicals and/or enhance the movement of current through the matrix. The field may also be utilized to control the flow or release of pharmacologically active agents and/or energy in a particular direction, thereby concentrating the pharmaceutical release to a predesignated location.

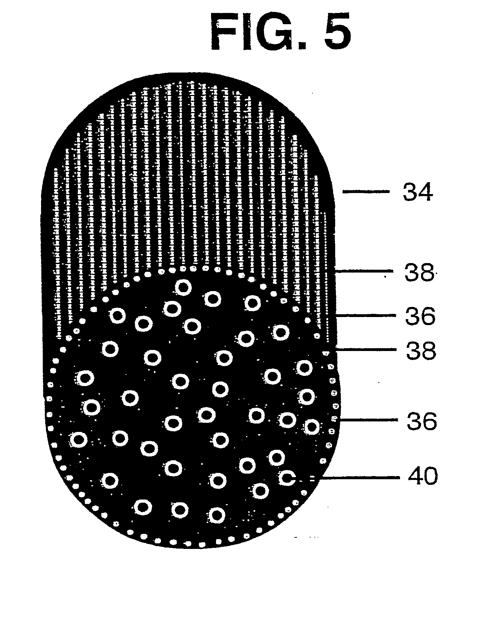

One embodiment of a drug delivery device which utilizes an electrical field to direct and control the release of one or more pharmacologically active agents is depicted in Figure 5. In Figure 5 an electrical current can travel through the conductive material 36 releasing the pharmacologically active agent(s) 40 from the protein matrix material 38 in the direction traveled by the current flow.

Cells associated with the protein matrix material as previously disclosed can be stimulated by electrically to release biochemicals. For example, natural or genetically engineered adrenal chromafin cells can be stimulated electrically to release therapeutic opiates, monoamines such as dopamine and other beneficial biochemicals.

Additionally, as previously described the matrix may include secondary migration sensitive devices including pharmacologically active agents that are in the form of microspheres, liposomes, lipospheres, particles and other types of vesicles. The flow of electrical current can stimulate the release of such devices by the heat generated. Many such migration sensitive drug delivery devices possess a lipid, oil or wax form and thereby are susceptible to melting and enhanced release when heated. Therefore, the transmission of an electrical current through the protein matrix material heats the protein matrix material and the embedded or suspended migration sensitive drug delivery devices, thereby melting the migration sensitive devices and releasing the pharmacologically active agents.

Additionally, the systematic, sustainable and/or consistent release of the drug delivery device may be attributed to the cohesive and interaction features present in the drug delivery device. As previously described, the protein matrix is compressed to eliminate part or all of the bulk water present in the cohesive body. This compression also compels and influences additional attracting forces amongst the protein molecules, solvent molecules and pharmacologically active agent molecules included in the matrix that would not be found if compression was not undertaken. Also other optional biocompatible materials, if included in the matrix, will be compelled and influenced to interact with the pharmacologically active agents to augment their release characteristics. This additional binding characteristic provides for a more systematic and controllable release of the pharmacologically active agents that are either trapped by interacting protein, optional biocompatible material and solvent molecules or that are also interacting with the protein, optional biocompatible material and solvent molecules themselves. Augmentation may include inhibiting or enhancing the release characteristics of the pharmacologically active agent(s). For example, a multi-layered drug delivery device may comprise alternating layers of protein matrix material that have sequential inhibiting and enhancing biocompatible materials included, thereby providing a pulsing release of pharmacologically active agents. A specific example may be utilizing glutamine in a layer as an enhancer and polyanhydride as an inhibitor. The inhibiting layer may include drugs or no drugs.

As previously suggested, embodiments of the drug delivery devices, produced and administered utilizing the methods of the present invention, are capable of the sustainable, controllable local delivery of pharmacologically active agent(s), while also providing the advantage of being capable of being degraded, and preferably safely resorbed and/or remodeled into the surrounding host tissue. The resorbable characteristic of various embodiments of the present invention eliminates the need for the removal of the drug delivery device from the patient once the pharmacologically active agent(s) have been completely delivered from the matrix. Alternatively, the drug delivery device may be produced to remain in the patient and provide a systematic and controllable diffusion of the pharmacololgically active agent(s) as described and suggested previously.

The drug delivery device of present invention may be formed into any shape and size, such as a cylinder, a tube, a wafer, particles or any other shape that may optimize the delivery of the incorporated pharmacologically active agent.

The delivery of electricity to various medical devices and materials is well known in the art. Any such delivery process, device or assembly my be utilized with embodiments of the present invention. These delivery means include but are not limited to the delivery of electricity to the drug deliver device and also all other protein matrix devices of the present invention by an electrical source that is incorporated within the device, that is implanted and placed within close proximity of the device, that is positioned outside the implanted tissue and connected by an electrical lead, that is transmitted through the tissue to a receiver or that supplies electricity in any other manner that would provide sufficient electricity to the protein matrix device.

### ELECTROMATRIX DEVICES

The delivery of electrical energy to portions of the human body has many beneficial and therapeutic attributes. As previously described the utilization of electrical energy can be combined with the protein matrix material of the present invention and pharmacologically active agents to assist in the administration of the pharmacologically active agents. Also, the delivery of electrical energy may be utilized to assist in providing heat treatment, ionization treatment and/or magnetic or electrical field treatment to various injured or diseased tissues or bones of the human body. For example the administration of electricity to an injured tissue or bone may assist in stimulating cell activity and/or attract ions (such as attraction of Ca⁺⁺ to injured bone) that could enhance the healing process.

The protein matrix material of the present invention also provides an appropriate environment for the embedding or adjoining of electric material within the solid protein matrix. Generally, protein matrix material, the same material as previously described, is integrated with materials that will conduct electricity instead of impregnated with or in combination with chemicals or pharmaceuticals. These conductive materials can be particles, fibers, patches wires, strands or any other form of conductive material which would then be insulated by the protein matrix to allow electricity to follow along the conductive material and throughout the protein matrix material.

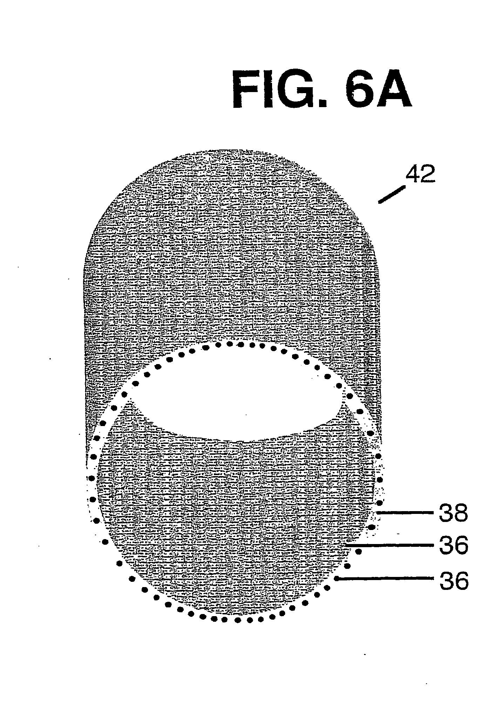

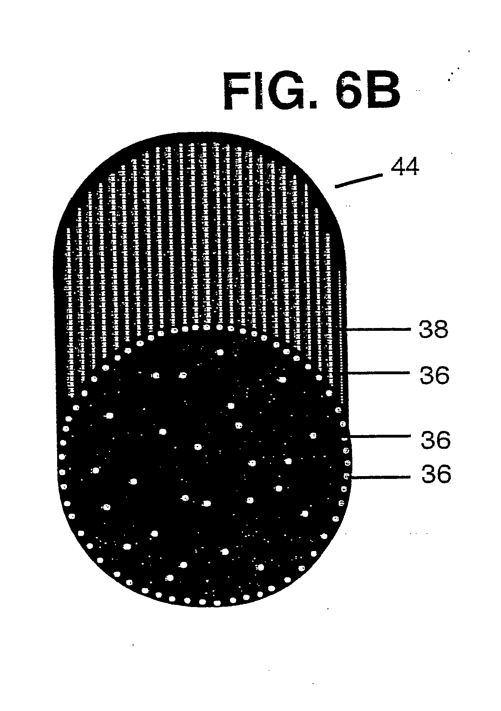

Figures 6A and 6B depict two embodiments of the protein matrix device of the present invention wherein the protein matrix material includes a conductive material. Figure 6A depicts an electromatrix device 42 that is comprised of tube of protein matrix material 38 that includes a plurality of conductive wires 36 embedded within. Figure 6B is a electromatrix device 44 that is formed in the shape of a cylinder. Similar to the embodiment of Figure 6A, the electromatrix cylinder 44 includes a protein matrix material 38 that includes a plurality of conductive wires 36. It is noted that the conductive material utilized in the electromatrix devices of the present invention may include any type and form of conductive material that would provide for the efficient and therapeutic delivery of electrical energy. The electromatrix device 42, 44 may be utilized to deliver electricity to tissue and/or bone to assist in the healing process, act as a functional stimulator and inhibit or facilitate metabolic activity. For example, the electromatrix may facilitate migration of Calcium ions to bone repair sites. An additional example is an electromatrix device that may be utilized to stimulate heart rhythm when adjoined to the corresponding medical device.

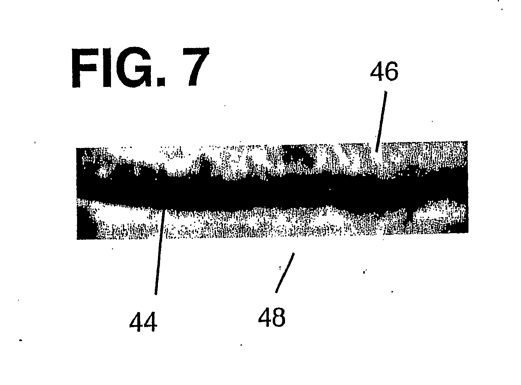

Figure 7 depicts another embodiment of the present invention wherein a conductive wire or cable 44 is embedded in a protein matrix material to form a receiver or antennae 48. The antennae 48, as depicted in Figure 7, implanted within the body would be very useful for telemetry or the transmission of energy to an implanted medical device. For example, a pacemaker implanted within the body that includes an antennae 48, as shown in Figure 7, may allow an individual to download data regarding new specifications for the pacemaker's operation or receive data regarding the functioning of the pacemaker or patients vital signs. Also, a longer antennae 48 would reduce the power required to perform such functions with a pace-maker located relatively deep within the body. Additionally, the internal power source of the pacemaker may be replenished by transmission of energy to the receiver or antennae 48 thereby providing for a power source that is not large and a system that would be able to operate for a longer period of time.

Furthermore, the incorporation of such an antennae 48 covered by the protein matrix material 46 of the present invention, as depicted in Figure 7, and incorporated into the patient's system would allow for the transmission of anything from a recalibration of the implanted electronic device to a complete download of a new operating program for the electronic device.

As previously suggested, the antennae 48 would also be able to transmit information from the electronic device to an apparatus outside the individual. Therefore, data regarding a patients vital signs to the actual condition of the electronic device could be transmitted out of the patient's body. Examples of the electronic device include a set up to take measurements, which would be important to the health of that patient, such as blood pressure, the temperature of the internal environment, impedance measurements which might be important to the functioning of the pace-maker, and/or similar types of data. This information could be read to make the decision on what type of calibrations that might be needed to the pacemaker, or whether the individual is suffering from some other type of problem or problems.

The protein matrix devices of the present invention, by having an affinity to electrical activity through the matrix, may be used as a lead; a biocompatible lead, operating so that electricity flows through the conductive material included in the lead, could be insulated to protect cells in the body that come in contact with that conductive material. This type of lead allows cells to integrate naturally with the protein matrix, but still allow electric current to pass through conductive material. The advantage of this would be a more biocompatible implant material that could be used as a delivery device of electrical current to tissue sites that are susceptible to inflammation or to current.

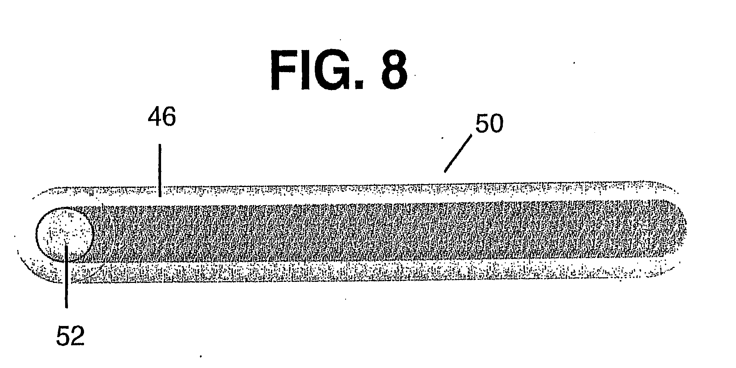

Figure 8 depicts a lead 50 such as a cable or wire 52 that is embedded within a protein matrix material 46. Similar to the antennae, the protein matrix material 46 may be utilized as an insulator so that the wire or cable 52 can extend through the host tissue and wherein the protein matrix material 46 acts as a biocompatible shield for the host tissue from the wire or cable 52. The lead 50 may operate as a conduit for electrical current between medical devices or between a medical device and a receiving tissue, such as a pacemaker electrically adjoined to heart tissue.

The leads of the present invention provides a mechanism for incorporating two or more devices within an individual which can work in conjunction with each other and transmit information back-and-forth from each device. Such a system could then also transmit to devices outside the patient through leads thereby allowing the transmitted data to be read by a person monitoring the patient while the device is continuously working within the patient.

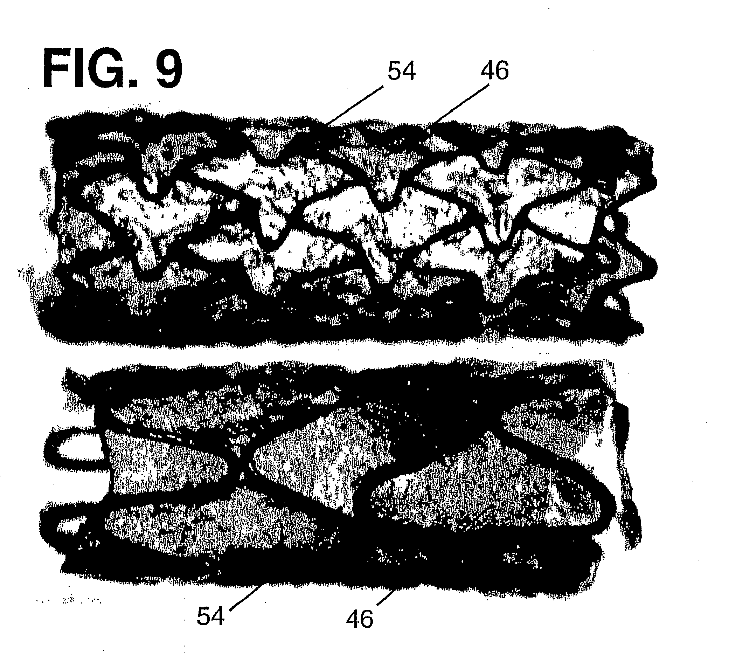

Other embodiments of the present invention include the utilization of the protein matrix material to encapsulate meshes or screens, such as or similar to stents. Figure 9 depicts two embodiments of the present invention wherein a screen or mesh is embedded, covered or masked by a protein matrix material 46. As depicted in Figure 9, the protein covered mesh may be formed in a tube or cylinder. However, the protein covered mesh may be of any shape and size, such as planar or angled, so as to adapt to the tissue or surface it is applied to. The protein matrix covered mesh of the present invention may be utilized for a number of beneficial biological functions. For example, an electrically charged protein matrix covered mesh may be utilized to attract certain compounds, chemicals or cells that may facilitate a desired function. Additionally, the protein charged mesh may be utilized to deliver an electrical charge to desired part of the body in the form of an electrode.

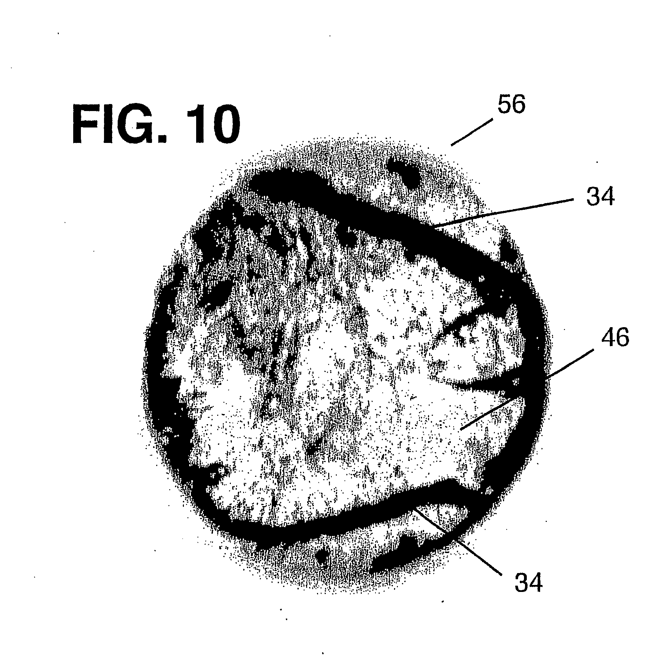

Figure 10 depicts another embodiment of the protein matrix devices of the present invention in the form of an electrode 56. Generally, the electrode 56 comprises a protein matrix material 46 that includes a plurality of conductive wires 34 and/or a conductive mesh material (not shown). The electrode 56 has a number of applications including, but not limited to electrocardiogram (ECG) applications and (electroencephalogram) EEG applications. An electrical current or transmission may be applied by the electrode 56 transdermally or parenterally by adjoining the electrode to the skin or implanting the electrode to the desired internal tissue.

Encapsulation of a mesh or screen with the protein matrix material of the present invention produces a protein matrix device that is more biocompatible with the host tissue than the mesh or screen alone. Such encapsulation or coating of the mesh or screen reduces or prevents adverse immuno-response reactions and inflammatory response to the protein matrix covered mesh or screen being administered and further enhances acceptance and remodeling of the protein matrix covered mesh or screen by the host tissue. Furthermore, encapsulated or coated protein matrix covered meshes or screens may also include one or more pharmacologically active agents, such as heparin, within or attached to the protein matrix material that may assist in the facilitation of tissue acceptance and remodeling as well as inhibit additional adverse conditions sometimes related to implantation of such devices. In addition to anti-platelet aggregation drugs, anti-inflammatory agents, gene altering agents such as antisense, and other pharmacologically active agents can be administered locally to the host tissue through the protein matrix material utilized with such devices.

The protein matrix material may completely encapsulate or otherwise coat the exterior of the stent. Generally, the encapsulated or coated protein matrix mesh or screen is made in a similar process as described above. The protein matrix covered meshes or screens, either encapsulated or coated generally have a wall thickness of approximately 0.05mm to 1 cm and preferably has a wall thickness of .15 to 0.50 cm.

As previously described additional polymeric and other biocompatible materials may be included in the protein matrix material to provide additional structural stability and durability to the encapsulated or coated protein matrix meshes. Also, other structural materials, such as proteoglycans, can be used in this process to add greater tissue imitation and biocompatibility. The proteoglycans can replace or be mixed with the protein material in the production of the protein matrix material.

Additionally, the protein matrix material included in the encapsulated or coated protein matrix mesh or screen may be cross-linked to provide additional desirable features such as the inhibition of cell growth or to provide additional structural durability and stability. For example, the protein matrix material of the encapsulated or coated protein matrix mesh may be crosslinked by contacting the material with a chemical reagent, such as glutaraldehyde, or other type of crosslinking reagent.

The embodiments illustrated in Figure 7, 9 and 10 include a protein matrix material comprising a 4:1 ratio of collagen to elastin. However, other embodiments of the present invention, may be produced by preparing a protein matrix device that includes ratios, combinations and types of proteins. For example, other protein matrix devices may be prepared by utilizing the following types of proteins and ratio of proteins: 2:1:2 collagen to elastin to albumen, 4:1 collagen to elastin, 1:4:15 heparin to elastin to collagen, 1:4:15 condroitin to elastin to collagen.

### COATINGS FOR IMPLANTABLE MEDICAL DEVICES

The protein matrix material of the present invention may also be utilized for coating of implantable medical devices. As previously described and similar to the other protein matrix devices of the present invention, the protein matrix material for the coating of implantable devices has the advantage of being capable of being safely resorbed and remodeled by the host tissue. The resorbable and remodeling characteristics of the protein matrix coated implantable medical devices allows for enhanced biocompatibility with the host tissue and a reduction in inflammatory response by the surrounding tissue.

The coated medical devices may be prepared as previously described by including the implantable medical device in the cohesive body, placing the cohesive body and medical device in a compression device and compressing them until a protein matrix coating is formed around the implantable medical device. Alternatively, the medical device may be coated by a sheet of premade protein matrix material that was produced by compressing the cohesive body without the medical device. The sheet of protein matrix material may be wrapped around the medical device and secured to itself by pressure or fastened to the device by suture, staples, adhesives or any other fastening means.

Any implantable medical devices that is covered or may be covered by a coating may be utilized in the present invention. Implantable medical devices that may be coated with the protein matrix material of the present invention include, but are not limited to, heart pumps, drug delivery pumps, pacemakers, defibrillators, catheters, hearing aids, imaging and diagnostic devices, biosensor devices, Micro-Electronics Miniaturized Systems (MEMS) such as a Peltier device, prosthetics such as hip, bone, penile, breast, knee, elbow and the like and any other device that may be implanted in the body of a patient.

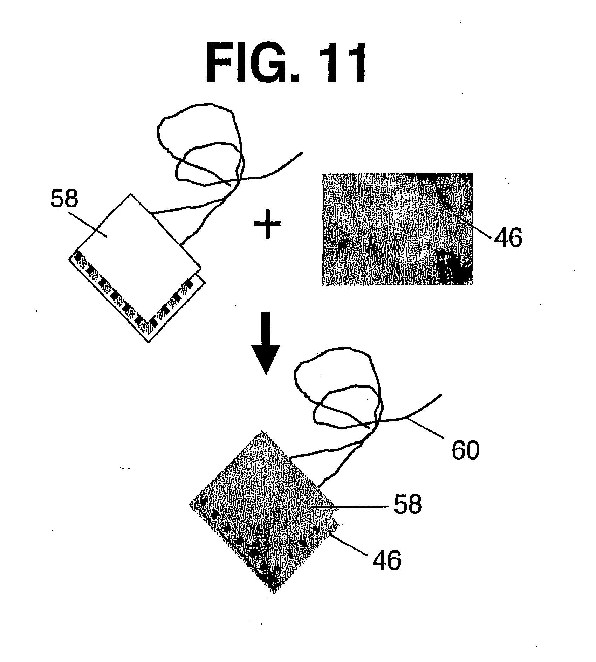

Figure 11 depicts one embodiment of an implantable medical device that is coated with a protein matrix material. As illustrated in Figure 11, an implantable device 58 is covered, masked or coated with a protein matrix material 46 as previously described. The protein matrix coated medical device may be attached to a lead 60, which is operably connected to a power source. Alternatively, the implantable medical device may include an internal power source or receiver or antennae that may receive energy from an outside source.

### IMAGING, IMPRINTED AND DIAGNOSTIC DEVICES

The protein matrix can have incorporated into it a marker or imaging system that allows the matrix to be located and imaged using ultrasound, MRI, X-Ray, PET or other imaging techniques. The materials incorporated into the protein matrix material of the present invention may be metallic, gaseous or liquid in nature. For example, the protein matrix may include contrast dyes or agents such as gadolinium gadopentetate dimeglumine (Gd-DPTA) for MRI imaging or may be made with air bubbles or density materials that allow easy visualization of the protein matrix by ultrasound. Furthermore, it may be possible to cause the material to react to an imaging technique, i.e., ultrasound to make bubbles or through the addition of another chemical or substance to the system such as an enzyme (e.g., peroxide addition to a protein matrix that contains peroxidase as an intrauterine marker can be monitored by ultrasound). Finally, liposomes that include a chemical containing iron may be utilized to enhance imaging of a particular area.

Another embodiment of the present invention is a protein matrix, which can include imprints that provide for specific site location for attachment of substances, such as chemicals, oligomers, cells or enzymes, or for preventing or reducing attachment of substances. Examples of cells, which may be targeted for specific attachment sites on the protein matrix may be cell adhesion molecules or electro-conductive molecules.

The protein matrix can be of any size, shape or form and can be imprinted with any pattern desired depending upon the application. For example, an embodiment of the imprinted protein matrix may take the form of a blood vessel. The exterior of the blood vessel may be imprinted with a pattern that limits the attachment of cellular material, which facilitates capillary growth to the exterior. This limitation of angiogenesis provides a number of benefits including the reduction of inflammation to the vessel surroundings.

Another embodiment includes the protein matrix in the form of a sphere. Such a matrix may be imprinted in areas, which are not intended to bind to biological tissue upon implantation. More specifically, a protein matrix may be impregnated with an adhesive substance, which would facilitate binding to tissue. Portions of the protein matrix not intended to bind to the tissue may be imprinted thereby preventing contact of the tissue with the adhesive substance.

Methods of imprinting the protein matrix with a desired pattern can be performed by any means known in the art such as chemical crosslinking with reagents such as glutaraldehyde, UV crosslinking or other crosslinking processes known in the art. For example, the utilization of UV light can produce a crosslinking pattern upon the protein matrix by including UV reacting reagents within the protein matrix material and exposing the protein matrix to UV light. One function of such a crosslinking pattern would be to inhibit the attachment of cells, chemicals, enzymes or other matter to the crosslinked areas of the protein matrix.

Another embodiment of the present invention relating to an imprinting method is the use of masking systems to create the imprinted pattern. The pattern on a protein matrix may be produced by covering the protein matrix with a mask that has the desired pattern and exposing the covered matrix to a chemical substance, such as glutaraldehyde or other crosslinking device. The portions of the protein matrix not covered by the mask are contacted by the chemical substance and crosslinking occurs. The mask is then removed thereby providing a protein matrix with both crosslinked and non-crosslinked portions. The non-crosslinked areas can provide locations for the attachment or access to chemicals, cells, enzymes, oligonucleotides or other proteins. These site specific attachment areas of the protein matrix may be utilized for diagnostic purposes like in array plates, the growth of cells or as access points for other chemicals or enzymes.

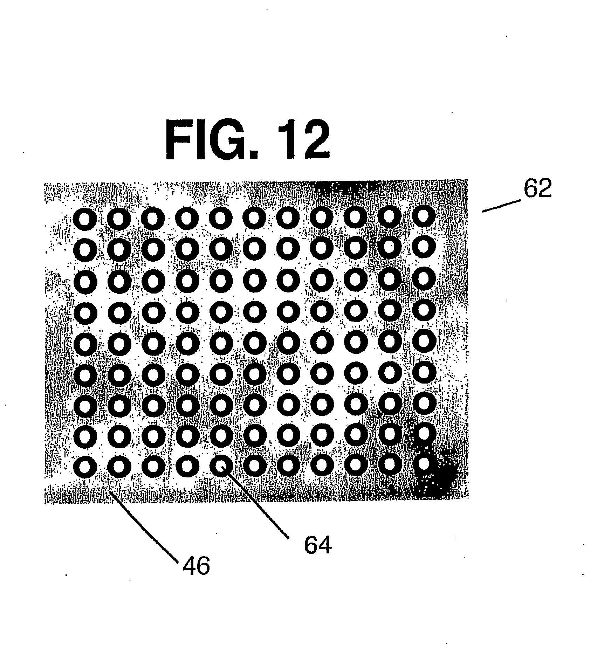

Figure 12 depicts an embodiment of a protein matrix diagnostic array plate 62. The array plate comprises a protein matrix 48 that may be crosslinked utilizing the above described masking and crosslinking techniques to produce attachment sites 64. An array may include 1 or more sites. The protein matrix 46 may cover a substrate such as glass or any other rigid material or may be formulated to create the plate without a substrate.

In one embodiment of the array plate of the present invention liver enzymes are included in the protein matrix of the array plate. The liver enzymes may be segregated into wells, such as the wells depicted in Figure 12, or they may be distributed homogeneously throughout the matrix. Potential pharmaceutical compounds or compositions that are used systemically may be tested for biological activity by washing or incubating solutions of such compounds or compositions over or with the array plate to test their interaction with the enzymes and to determine their metabolism with the enzymes. Testing of the solutions after washing over the array plate may be performed utilizing many different methods known in the art, such as spectrophotometry, gas chromatography, liquid chromatography and the like.

Also, a protein matrix may be set up as a two enzyme array wherein the enzymes may be separated or homogenously distributed throughout the protein matrix material. For example, it is important to address the clearance of urea in some patients. Inability to clear urea can lead to hyperammoneimia (toxic) and can occur during liver failure and possibly renal disease. A UV-based urea nitrogen assay is as follows:

urea + H2O ---UREASE---> 2NH3 + CO2

NH3 + 2-oxoglutarate + NADH ---glutamate dehydrogenase----> glutamate + NAD + H2O

Therefore, urease and glutamate dehydrogenase incorporated into the protein matrix is used to test for urea by washing over or incubating test samples with this type of protein matrix array. Testing of these samples for the presence and amount of enzymatic activity after washing over the array plate may be performed utilizing many different methods known in the art, such as spectrophotometry, gas chromatography, liquid chromatography and the like.

Additionally, the protein matrix array plates of the present invention may include indicators within the matrix that would provide an optical, visual, imaging or other type of indication that a substance is present or that enzyme or chemical activity occurred at the site. Alternatively, the sites may also be reactive to an indicator applied to them that would provide an optical, visual, imaging or other type of indication that a substance is present or that enzyme or chemical activity occurred at the site.

Finally the imprinted protein matrix has applications in the protein chip technology described above. The imprinting of patterns upon the protein matrix chip may produce chips, which provide a number of similar characteristics as a silicon chip.

### EXAMPLES

The protein matrix devices of the present invention will now be further described with reference to the following non-limiting examples and the following materials and methods that were employed.

### Example #1: Preparation of Collagen:Elastin (4:1 ratio) Protein Matrix Antennae:

In the preparation of the protein matrix antennae as illustrated in Figure 7, Collagen:Elastin was used in a 4:1 ratio and mixed with sterilized saline in amount equal to 600% the weight of the combined collagen and elastin (e.g., 80mg collagen + 20mg elastin in 600microliters of water). The material was mixed together and immediately thereafter, the pH was adjusted with drops of 0.1N and 0.5N NaOH until pH indicator strips read 7.4 pH. The material was then partially dried at room temperature until it was to a state where it was cohesive unto itself and was then subsequently formed into a cohesive body. The conductive microgauge wire was loaded into the mold by wrapping the wire around the mold insert to expose a portion of wire to the cohesive body that was subsequently loaded. Mechanically applied pressure forced the cohesive body over and around the wire with a final pressure equal to 4,000 psi for a period of 2 hours. The result was the formation of an antennae including a protein matrix coating have a protein matrix thickness of approximately 0.2 mm and the length of the protein matrix antennae was approximately 1 cm. The protein matrix antennae was then slipped off the insert and stored in a clean dry plastic tube.

### Example #2: Preparation of Collagen:Elastin (4:1 ratio) Protein Matrix Meshes:

In the preparation of the protein matrix meshes as illustrated in Figure 9, Collagen:Elastin was used in a 4:1 ratio and mixed with sterilized saline in amount equal to 600% the weight of the combined collagen and elastin (e.g., 80mg collagen + 20mg elastin in 600microliters of water). The material was mixed together and immediately thereafter, the pH was adjusted with drops of 0.1N and 0.5N NaOH until pH indicator strips read 7.4 pH. The material was then partially dried at room temperature until it was to a state where it was cohesive unto itself and was then subsequently formed into a cohesive body. The conductive stainless steel mesh was loaded into the mold where a mandrel insert received the mesh by wrapping the mesh around it and the cohesive body was then subsequently loaded. Mechanically applied pressure forced the cohesive body over the mandrel and around the mesh with a final pressure equal to 5,000 psi for a period of 10 minutes. The result was the formation of a protein matrix coated mesh formed around the mandrel where the protein matrix mesh wall thickness was 0.2 mm and the length of the protein matrix mesh was approximately 1 cm. While the protein matrix mesh was still on the mandrel insert, it was submersed in 1% glutaraldyhde solution for 2 minutes, resulting in partial cross linking of the outside of the protein matrix mesh. After 2 minutes, the mesh-mandrel insert was submersed in saline for 1 minute then it was subjected to a 15 minute submersion in a 0.1 M phosphate buffered saline solution containing 1% glutamine and 1% glycine. The protein matrix mesh was then slipped off the mandrel, where the mandrel was made with a slope of .001 inches over the 1 cm length to ease the removal of the protein matrix mesh. Also, before the mandril was placed in the mold it was coated with a slippery substance (e.g., glycol or Triton-X100). Finished protein matrix meshes were stored in saline and sterilized with 10-20 KRADS of gamma irradiation from a cesium source.

### Example #3: Preparation of Collagen:Elastin (4:1 ratio) Protein Matrix Electrodes:

In the preparation of the protein matrix electrodes as illustrated in Figure 10, Collagen:Elastin was used in a 4:1 ratio and mixed with sterilized saline in amount equal to 600% the weight of the combined collagen and elastin (e.g., 80mg collagen + 20mg elastin in 600microliters of water). The material was mixed together and immediately thereafter, the pH was adjusted with drops of 0.1N and 0.5N NaOH until pH indicator strips read 7.4 pH. The material was then partially dried at room temperature until it was to a state where it was cohesive unto itself and was then subsequently formed into a cohesive body. The conductive wires or mesh was loaded into the mold and wrapped around and insert exposing the wires or mesh to the subsequently loaded cohesive body. Mechanically applied pressure forced the cohesive body over and around the wires or mesh with a final pressure equal to 4,000 psi for a period of 120 minutes. The result was the formation of a protein matrix coated electrode formed around the insert where the protein matrix electrode had a wall thickness of approximately 03 mm and the diameter of the protein matrix mesh was approximately 1 cm. The protein matrix electrode was then removed from the insert and stored in a dry plastic tube.

### Example 4: Preparation of a Protein Matrix Array Comprising a Biodegradable Protein and an Enzyme:

The enzyme xanthine oxidase was dissolved in deionized water to 0.28 units/100 µl. This xanthine oxidase solution was mixed in with 50 mg protein (SELP 7) to form a coatable composition. The composition was then coated on a glass surface to form a film with a thickness of from about 0.1 to about 0.3 mm. The coated film was allowed to dry at room temperature until dry enough so as to be cohesive, i.e., to a solvent content of from about 50% to about 70%. The resulting film was rolled up, placed in a 3.5 mm diameter mold and compressed at 1750 psi for 2 minutes to form a 3.5 mm diameter cylinder, approximately 5 mm long, utilizing the compression molding device discussed hereinabove. The resulting cylinder had a solvent content of approximately 30% to about 60%. This cylinder was cut into four equal wafers so that each piece contained approximately 0.07 xanthine oxidase units/piece. These wafers were frozen at -80°C until used within 4 weeks.

### Example 5: Preparation of a Protein Matrix Array Comprising a Biodegradable Protein and an Enzyme:

The enzyme superoxide dismutase (SOD) was dissolved in deionized water to 30.0 units/100 µl. This SOD solution was mixed with 50 mg (SELP7) to form a coatable composition. The composition was then coated on a glass surface to form a film with a thickness of from about 0.1 mm to about 0.3 mm. The coated film was allowed to dry at room temperature until dry enough so as to be cohesive, i.e., to a solvent content of from about 50% to about 70%. The resulting film was rolled up, placed in a 3.5 mm diameter mold and compressed at 1750 psi for 2 minutes to form a 3.5 mm diameter cylinder, approximately 5 mm long, utilizing the compression molding device discussed hereinabove. The resulting cylinder had a solvent content of from about 30% to about 60%. This cylinder was cut into four equal wafers so that each piece contained approximately 7.5 units of SOD per /piece. These wafers were frozen at -80°C until used within 4 weeks.

### Example 6: In vitro Experiment with a Protein Matrix Array Comprising a Biodegradable Protein and an Enzyme:

A single cylinder piece, prepared as described above in Example 4, was added to a reaction chamber in a spectrophotometer containing xanthine, cytochrome C and other reactants according to previously described superoxide dismutase protocol (Sigma Quality Control Test Procedure EC 1.15.1.1 "Enzymatic Assay of Superoxide Dismutase") enzyme activity of the enzyme xanthine oxidase in the piece was calculated at 0.0005 delta absorbance min (absorbance measured at 550 mm where no enzyme activity produces 0.00000 change in absorbance). In comparison to a 0.01 unit solution of xanthine oxidase, which produced 0.0250 delta absorbance/min, the activity of the xanthine oxidase in the piece equaled 1% of the control solution in a time period of only 3 minutes. Thus, this result indicates that the diffusional barrier provided by the biodegradable polymeric matrix of the protein matrix array allows the enzyme to remain active from within the protein matrix array.

### Example 7: In Vitro Experiment with a Protein Matrix Array Comprising a Biodegradable Protein and an Enzyme:

In this assay system, xanthine oxidase, xanthine, cytochrome C and other reactants were added together to produce a delta absorbance of 0.0250/min. (Sigma Quality Control Test Procedure EC 1.15.1.1 "Enzymatic Assay of Superoxide Dismutase"). SOD activity is measured as the inhibition of the rate of reduction of ferricytochrome C by superoxide, observed at 550 nm, as described by J. McCord, I. J. Biol Chem., 244, 6049 (1969). The addition of a SOD containing piece, produced as described in Example 5 hereinabove, reduced the reaction to 0.0233 delta absorbance/min. Since 1 unit SOD will inhibit the reaction of cytochrome C by 50% in a coupled system using xanthine oxidase, it can be determined that the activity of the SOD pellet equaled 0.14 units of SOD. This activity represents about 2% of the SOD loaded into the biodegradable protein matrix of the protein matrix array. Thus, this result indicates that the diffusional barrier provided by the biodegradable polymeric matrix of the protein matrix array allows the enzyme to remain active from within the protein matrix array.

While the invention has been described in conjunction with specific embodiments thereof, it is evident that many alternatives, modifications, and variations will be apparent to those skilled in the art in light of the foregoing description. Accordingly, it is intended to embrace all such alternatives, modifications, and variations, which fall within the spirit and broad scope of the invention.

The invention will now be described by way of a series of numbered clauses.
1. A current released drug delivery device comprising one or more biocompatible protein materials, one or more conductive materials, one or more pharmacologically active agents and one or more biocompatible solvents, wherein the protein materials, conductive materials, pharmacologically active agents and biocompatible solvents are compressed to remove bulk biocompatible solvent and generate additional interactive forces to form the current released drug delivery device.
2. The current released drug delivery device of clause 1 wherein the biocompatible proteins may be natural, synthetic or genetically engineered.
3. The current released drug delivery device of clause 2 wherein the biocompatible proteins are natural proteins selected from the group consisting of elastin, collagen, albumin, keratin, fibronectin, silk, silk fibroin, actin, myosin, fibrinogen, thrombin, aprotinin and antithrombin III.
4. The current released drug delivery device of clause 2 wherein the biocompatible proteins are genetically engineered proteins made of blocks selected from the group consisting of elastinlike blocks, silklike blocks, collagenlike blocks, lamininlike blocks, fibronectinlike blocks and silklike and elastinlike blocks.
5. The current released drug delivery device of clause 1 wherein the biocompatible solvent is selected from the group consisting of water, dimethyl sulfoxide (DMSO), biocompatible alcohols, biocompatible acids, oils and biocompatible glycols.
6. The current released drug delivery device of clause 5 wherein the biocompatible solvent is water.
7. The current released drug delivery device of clause 1 wherein the one or more pharmacologically active agents are selected from the group consisting of analgesics, anesthetics, antipsychotic agents, steroids, antisteroids, corticosteroids, antiglacoma agents, antialcohol agents, anti-coagulants agents, genetic material, antithrombogenic agents, anticancer agents, anti-Parkinson agents, antiepileptic agents, anti-inflammatory agents, anticonception agents, enzymes agents, cells, growth factors, antiviral agents, antibacterial agents, antifungal agents, hypoglycemic agents, antihistamine agents, chemoattractants, neutraceuticals, antiobesity, smoking cessation agents, obstetric agents and antiasmatic agents.
8. The current released drug delivery device of clause 1, wherein the pharmacologically active agents comprises a second, migration-vulnerable drug delivery device.
9. The current released drug delivery device of clause 8, wherein the migration-vulnerable drug delivery device comprises a plurality of lipospheres homogeneously dispersed within the drug delivery device.
10. The current released drug delivery device of clause 8, wherein the migration-vulnerable drug delivery device comprises a plurality of microspheres homogeneously dispersed within the drug delivery device.
11. The current released drug delivery device of clause, 1, wherein the pharmacologically active agent is substantially homogeneously distributed within the drug delivery device.
12. The current released drug delivery device of clause 1 further comprising one or more biocompatible polymeric materials.
13. The current released drug delivery device of clause 12 wherein the one or more biocompatible polymeric materials are selected from the group consisting of epoxies, polyesters, acrylics, nylons, silicones, polyanhydride, polyurethane, polycarbonate, poly(tetrafluoroethylene), polycaprolactone, polyethylene oxide, polyethylene glycol, poly(vinyl chloride), polylactic acid, polyglycolic acid, polypropylene oxide, poly(akylene)glycol, polyoxyethylene, sebacic acid, polyvinyl alcohol, 2-hydroxyethyl methacrylate, polymethyl methacrylate, 1,3-bis(carboxyphenoxy)propane, lipids, phosphatidylcholine, triglycerides, polyhydroxybutyrate, polyhydroxyvalerate, poly(ethylene oxide), poly ortho esters, poly (amino acids), polycynoacrylates, polyphophazenes, polysulfone, polyamine, poly (amido amines), fibrin, graphite, flexible fluoropolymer, isobutyl-based, isopropyl styrene, vinyl pyrrolidone, cellulose acetate dibutyrate, silicone rubber, and copolymers of these.
14. The current released drug delivery device of clause 1 wherein the current released drug delivery device is crosslinked with one or more crosslinking agents.
15. The current released drug delivery device of clause 14 wherein the one or more crosslinking reagents are selected from the group consisting of glutaraldehyde, p-Azidobenzolyl Hydazide, N-5-Azido 2-nitrobenzoyloxysuccinimide, N-Succinimidyl 6-[4'azido-2'nitro-phenylamino]hexanoate and 4-[p-Azidosalicylamido] butylamine.
16. The current released drug delivery device of clause, 1 wherein the one or more conductive materials are selected from the group consisting of gold, silver, aluminum, platinum, tungsten, stainless steel, nitinol, copper, niobium, titanium, and ceramics
17. The current released drug delivery device of clause 1 wherein the one or more conductive materials comprises an alloy including one or more substances selected from the group consisting of gold, silver, tungsten, niobium, cobalt, titanium, zirconium, vanadium, molybdenum, nickel, iron, zinc, and copper.
18. A method of making a current released drug delivery device, comprising the steps of:
   (a) preparing a coatable composition including the one or more biocompatible protein materials, one or more conductive materials, one or more pharmacologically active agents and the one or more biocompatible solvents;
   (b) coating the composition to form a film;
   (c) partially drying the coated film until the coated film can be formed into a cohesive body;
   (d) forming said cohesive body; and compressing the cohesive body to form a current released drug delivery device.
19. The method of making a current released drug delivery device of clause 18 wherein the conductive materials are not added until the coated film is partially dried.
20. The method of making a current released drug delivery device of clause 18 wherein the biocompatible proteins may be natural, synthetic or genetically engineered.
21. The method of making a current released drug delivery device of clause 19 wherein the biocompatible proteins may be natural, synthetic or genetically engineered.
22. The method of making a current released drug delivery device of clause 20 wherein the biocompatible proteins are natural proteins selected from the group consisting of elastin, collagen, albumin, keratin, fibronectin, silk, silk fibroin, actin, myosin, fibrinogen, thrombin, aprotinin and antithrombin Ill.
23. The method of making a current released drug delivery device of clause 21 wherein the biocompatible proteins are natural proteins selected from the group consisting of elastin, collagen, albumin, keratin, fibronectin, silk, silk fibroin, actin, myosin, fibrinogen, thrombin, aprotinin and antithrombin Ill.
24. The method of making a current released drug delivery device of clause 20 wherein the biocompatible proteins are genetically engineered proteins made of blocks selected from the group consisting of elastinlike blocks, silklike blocks, collagenlike blocks, lamininlike blocks, fibronectinlike blocks and silklike and elastinlike blocks.
25. The method of making a current released drug delivery device of clause 21 wherein the biocompatible proteins are genetically engineered proteins made of blocks selected from the group consisting of elastinlike blocks, silklike blocks, collagenlike blocks, lamininlike blocks, fibronectinlike blocks and silklike and elastinlike blocks.
26. The method of making a current released drug delivery device of clause 18 wherein the biocompatible solvent is selected from the group consisting of water, dimethyl sulfoxide (DMSO), biocompatible alcohols, biocompatible acids, oils and biocompatible glycols.
27. The method of making a current released drug delivery device of clause 19 wherein the biocompatible solvent is selected from the group consisting of water, dimethyl sulfoxide (DMSO), biocompatible alcohols, biocompatible acids, oils and biocompatible glycols.
28. The method of making a current released drug delivery device of clause 26 wherein the biocompatible solvent is water.
29. The method of making a current released drug delivery device of clause 27 wherein the biocompatible solvent is water.
30. The method of making a current released drug delivery device of clause 18 wherein the one or more pharmacologically active agents are selected from the group consisting of analgesics, anesthetics, anti psychotic agents, steroids, antisteroids, corticosteroids, antiglacoma agents, antialcohol agents, anticoagulants agents, genetic material, antithrombolytic agents, anticancer agents, anti-Parkinson agents, antiepileptic agents, anti-inflammatory agents, anticonception agents, enzymes agents, cells, growth factors, antiviral agents, antibacterial agents, antifungal agents, hypoglycemic agents, antihistamine agents, chemoattractants, neutraceuticals, antiobesity, smoking cessation agents and antiasmatic agents.
31. The method of making a current released drug delivery device of clause 19 wherein the one or more pharmacologically active agents are selected from the group consisting of analgesics, anesthetics, anti psychotic agents, steroids, antisteroids, corticosteroids, antiglacoma agents, antialcohol agents, anticoagulants agents, genetic material, antithrombolytic agents, anticancer agents, anti-Parkinson agents, antiepileptic agents, anti-inflammatory agents, anticonception agents, enzymes agents, cells, growth factors, antiviral agents, antibacterial agents, antifungal agents, hypoglycemic agents, antihistamine agents, chemoattractants, neutraceuticals, antiobesity, smoking cessation agents and antiasmatic agents.
32. The method of making a current released drug delivery device of clause 18, wherein the pharmacologically active agent comprises a second, migration-vulnerable drug delivery device.
33. The method of making a current released drug delivery device of clause 19, wherein the pharmacologically active agent comprises a second, migration-vulnerable drug delivery device.
34. The method of making a current released drug delivery device of clause 32, wherein the migration-vulnerable drug delivery device comprises a plurality of lipospheres, micro spheres or a combination thereof homogeneously dispersed within the current released drug delivery device.
35. The method of making a current released drug delivery device of clause 33, wherein the migration-vulnerable drug delivery device comprises a plurality of lipospheres, microspheres or a combination thereof homogeneously dispersed within the current released drug delivery device.
36. The method of making a current released drug delivery device of clause 18, wherein the pharmacologically active agent is substantially homogeneously distributed within the current released drug delivery device.
37. The method of making a current released drug delivery device of clause 19, wherein the pharmacologically active agent is substantially homogeneously distributed within the current released drug delivery device.
38. The method of making a current released drug delivery device of clause 18 further comprising one or more biocompatible polymeric materials.
39. The method of making a current released drug delivery device of clause 19 further comprising one or more biocompatible polymeric materials.
40. The method of making a current released drug delivery device of clause 38 wherein the one or more biocompatible polymeric materials are selected from the group consisting of epoxies, polyesters, acrylics, nylons, silicones, polyanhydride, polyurethane, polycarbonate, poly(tetrafluoroethylene), polycaprolactone, polyethylene oxide, polyethylene glycol, poly(vinyl chloride), polylactic acid, polyglycolic acid, polypropylene oxide, poly(akylene)glycol, polyoxyethylene, sebacic acid, polyvinyl alcohol, 2-hydroxyethyl methacrylate, polymethyl methacrylate, 1,3-bis(carboxyphenoxy)propane, lipids, phosphatidylcholine, triglycerides, polyhydroxybutyrate, polyhydroxyvalerate, poly(ethylene oxide), poly ortho esters, poly (amino acids), polycynoacrylates, polyphophazenes, polysulfone, polyamine, poly (amido amines), fibrin, graphite, flexible fluoropolymer, isobutyl-based, isopropyl styrene, vinyl pyrrolidone, cellulose acetate dibutyrate, silicone rubber, and copolymers of these.
41. The method of making a current released drug delivery device of clause 39 wherein the one or more biocompatible polymeric materials are selected from the group consisting of epoxies, polyesters, acrylics, nylons, silicones, polyanhydride, polyurethane, polycarbonate, poly(tetrafluoroethylene), polycaprolactone, polyethylene oxide, polyethylene glycol, poly(vinyl chloride), polylactic acid, polyglycolic acid, polypropylene oxide, poly(akylene)glycol, polyoxyethylene, sebacic acid, polyvinyl alcohol, 2-hydroxyethyl methacrylate, polymethyl methacrylate, 1,3-bis(carboxyphenoxy)propane, lipids, phosphatidylcholine, triglycerides, polyhydroxybutyrate, polyhydroxyvalerate, poly(ethylene oxide), poly ortho esters, poly(amino acids), polycynoacrylates, polyphophazenes, polysulfone, polyamine, poly (amido amines), fibrin, graphite, flexible fluoropolymer, isobutyl-based, isopropyl styrene, vinyl pyrrolidone, cellulose acetate dibutyrate, silicone rubber, and copolymers of these.
42. The method of making a current released drug delivery device of clause 18 wherein the current released drug delivery device is crosslinked with one or more crosslinking agents.
43. The method of making a current released drug delivery device of clause 19 wherein the current released drug delivery device is crosslinked with one or more crosslinking agents.
44. The method of making a current released drug delivery device of clause 42 wherein the crosslinking agents are selected from the group consisting of glutaraldehyde, p-Azidobenzolyl Hydazide, N-5-Azido-2 nitrobenzoyloxysuccinimide, N-Succinimidyl 6-[4'azido-2'nitro-phenylamino]hexanoate and 4 [p-Azidosalicylamido] butylamine.
45. The method of making a current released drug delivery device of clause 43 wherein the one or more crosslinking reagents are selected from the group consisting of glutaraldehyde, p-Azidobenzolyl Hydazide, N-5-Azido 2-nitrobenzoyioxysuccinimide, N-Succinimidyl 6-[4'azido-2'nitro-phenylamino]hexanoate and 4-[p-Azidosalicylamido] butylamine.
46. The method of making a current released drug delivery device of clause 18 wherein the one or more conductive materials are selected from the group consisting of gold, silver, aluminum, platinum, tungsten, stainless steel, nitinol, copper, niobium, titanium, and ceramics.
47. The method of making a current released drug delivery device of clause 19 wherein the one or more conductive materials are selected from the group consisting of gold, silver, aluminum, platinum, tungsten, stainless steel, nitinol, copper, niobium, titanium, and ceramics.
48. The method of making a current released drug delivery device of clause 18 wherein the one or more conductive materials comprises an alloy including one or more substances selected from the group consisting of gold, silver, tungsten, niobium, cobalt, titanium, zirconium, vanadium, molybdenum, nickel, iron, zinc, and copper.
49. The method of making a current released drug delivery device of clause 19 wherein the one or more conductive materials comprises an alloy including one or more substances selected from the group consisting of gold, silver, tungsten, niobium, cobalt, titanium, zirconium, vanadium, molybdenum, nickel, iron, zinc, and copper.
50. An electromatrix device comprising one or more biocompatible protein materials, one or more conductive materials, zero or more pharmacologically active agents and one or more biocompatible solvents, wherein the protein materials, conductive materials, pharmacologically active agents and biocompatible solvents are compressed to remove bulk biocompatible solvent and generate additional interactive forces to form the electromatrix device.
51. The electromatrix device of clause 50 wherein the biocompatible proteins may be natural, synthetic or genetically engineered.
52. The electromatrix device of clause 51 wherein the biocompatible proteins are natural proteins selected from the group consisting of elastin, collagen, albumin, keratin, fibronectin, silk, silk fibroin, actin, myosin, fibrinogen, thrombin, aprotinin and antithrombin III.
53. The electromatrix device of clause 51 wherein the biocompatible proteins are genetically engineered proteins made of blocks selected from the group consisting of elastinlike blocks, silklike blocks, collagenlike blocks, lamininlike blocks, fibronectinlike blocks and silklike and elastinlike blocks.
54. The electromatrix device of clause 50 wherein the biocompatible solvent is selected from the group consisting of water, dimethyl sulfoxide (DMSO), biocompatible alcohols, biocompatible acids, oils and biocompatible glycols.
55. The electromatrix device of clause 54 wherein the biocompatible solvent is water.
56. The electromatrix device of clause 50 wherein the one or more pharmacologically active agents are selected from the group consisting of analgesics, anesthetics, antipsychotic agents, steroids, antisteroids, corticosteroids, antiglacoma agents, antialcohol agents, anti-coagulants agents, genetic material, antithrombogenic agents, anticancer agents, anti-Parkinson agents, antiepileptic agents, anti-inflammatory agents, anticonception agents, enzymes agents, cells, growth factors, antiviral agents, antibacterial agents, antifungal agents, hypoglycemic agents, antihistamine agents, chemoattractants, neutraceuticals, antiobesity, smoking cessation agents, obstetric agents and antiasmatic agents.
57. The electromatrix device of clause 50, wherein the pharmacologically active agents comprises a second, migration-vulnerable drug delivery device.
58. The electromatrix device of clause 57, wherein the migration-vulnerable drug delivery device comprises a plurality of lipospheres homogeneously dispersed within the electromatrix device.
59. The electromatrix device of clause 57, wherein the migration-vulnerable drug delivery device comprises a plurality of microspheres homogeneously dispersed within the electromatrix device.
60. The electromatrix device of clause 50, wherein the pharmacologically active agent is substantially homogeneously distributed within the electromatrix device.
61. The electromatrix device of clause 50 further comprising one or more biocompatible polymeric materials.
62. The electromatrix device of clause 61 wherein the one or more biocompatible polymeric materials are selected from the group consisting of epoxies, polyesters, acrylics, nylons, silicones, polyanhydride, polyurethane, polycarbonate, poly(tetrafluoroethylene), polycaprolactone, polyethylene oxide, polyethylene glycol, poly(vinyl chloride), polylactic acid, polyglycolic acid, polypropylene oxide, poly(akylene)glycol, polyoxyethylene, sebacic acid, polyvinyl alcohol, 2-hydroxyethyl methacrylate, polymethyl methacrylate, 1,3-bis(carboxyphenoxy)propane, lipids, phosphatidylcholine, triglycerides, polyhydroxybutyrate, polyhydroxyvalerate, poly(ethylene oxide), poly ortho esters, poly (amino acids), polycynoacrylates, polyphophazenes, polysulfone, polyamine, poly (amido amines), fibrin, graphite, flexible fluoropolymer, isobutyl-based, isopropyl styrene, vinyl pyrrolidone, cellulose acetate dibutyrate, silicone rubber, and copolymers of these.
63. The electromatrix device of clause 50 wherein the current released drug delivery device is crosslinked with one or more crosslinking agents.
64. The electromatrix device of clause 63 wherein the one or more crosslinking reagents are selected from the group consisting of glutaraldehyde, p-Azidobenzolyl Hydazide, N-5-Azido 2-nitrobenzoyloxysuccinimide, N-Succinimidyl 6-[4'azido-2'nitro-phenylamino]hexanoate and 4-[p-Azidosalicylamido] butylamine.
65. The electromatrix device of cause 50 wherein the one or more conductive materials are selected from the group consisting of gold, silver, aluminum, platinum, tungsten, stainless steel, nitinol, copper, niobium, titanium, and ceramics
66. The electromatrix device of clause 50 wherein the one or more conductive materials comprises an alloy including one or more substances selected from the group consisting of gold, silver, tungsten, niobium, cobalt, titanium, zirconium, vanadium, molybdenum, nickel, iron, zinc, and copper.
67. A method of making an electromatrix device, comprising the steps of:
   (a) preparing a coatable composition including the one or more biocompatible protein materials, one or more conductive materials, one or more pharmacologically active agents and the one or more biocompatible solvents;
   (b) coating the composition to form a film;
   (c) partially drying the coated film until the coated film can be formed into a cohesive body;
   (d) forming said cohesive body; and compressing the cohesive body to form an electromatrix .
68. The method of making an electromatrix device of clause 67 wherein the conductive materials are not added until the coated film is partially dried.
69. The method of making an electromatrix device of clause 67 wherein the biocompatible proteins may be natural, synthetic or genetically engineered.
70. The method of making an electromatrix device of clause 68 wherein the biocompatible proteins may be natural, synthetic or genetically engineered.
71. The method of making an electromatrix device of clause 69 wherein the biocompatible proteins are natural proteins selected from the group consisting of elastin, collagen, albumin, keratin, fibronectin, silk, silk fibroin, actin, myosin, fibrinogen, thrombin, aprotinin and antithrombin Ill.
72. The method of making an electromatrix device of clause 70 wherein the biocompatible proteins are natural proteins selected from the group consisting of elastin, collagen, albumin, keratin, fibronectin, silk, silk fibroin, actin, myosin, fibrinogen, thrombin, aprotinin and antithrombin Ill.
73. The method of making an electromatrix device of clause 69 wherein the biocompatible proteins are genetically engineered proteins made of blocks selected from the group consisting of elastinlike blocks, silklike blocks, collagenlike blocks, lamininlike blocks, fibronectinlike bloclcs and silklike and elastinlike blocks.
74. The method of making an electromatrix device of clause 70 wherein the biocompatible proteins are genetically engineered proteins made of blocks selected from the group consisting of elastinlike blocks, silklike blocks, collagenlike blocks, lamininlike blocks, fibronectinlike blocks and silklike and elastinlike blocks.
75. The method of making an electromatrix device of clause 67 wherein the biocompatible solvent is selected from the group consisting of water, dimethyl sulfoxide (DMSO), biocompatible alcohols, biocompatible acids, oils and biocompatible glycols.
76. The method of making an electromatrix device of clause 68 wherein the biocompatible solvent is selected from the group consisting of water, dimethyl sulfoxide (DMSO), biocompatible alcohols, biocompatible acids, oils and biocompatible glycols.
77. The method of making an electromatrix device of clause 75 wherein the biocompatible solvent is water.
78. The method of making an electromatrix device' of clause 76 wherein the biocompatible solvent is water.
79. The method of making an electromatrix device of clause 67 wherein the one or more pharmacologically active agents are selected from the group consisting of analgesics, anesthetics, anti psychotic agents, steroids, antisteroids, corticosteroids, antiglacoma agents, antialcohol agents, anticoagulants agents, genetic material, antithrombolytic agents, anticancer agents, anti-Parkinson agents, antiepileptic agents, anti-inflammatory agents, anticonception agents, enzymes agents, cells, growth factors, antiviral agents, antibacterial agents, antifungal agents, hypoglycemic agents, antihistamine agents, chemoattractants, neutraceuticals, antiobesity, smoking cessation agents and antiasmatic agents.
80. The method of making an electromatrix device of clause 68 wherein the one or more pharmacologically active agents are selected from the group consisting of analgesics, anesthetics, anti psychotic agents, steroids, antisteroids, corticosteroids, antiglacoma agents, antialcohol agents, anticoagulants agents, genetic material, antithrombolytic agents, anticancer agents, anti-Parkinson agents, antiepileptic agents, anti-inflammatory agents, anticonception agents, enzymes agents, cells, growth factors, antiviral agents, antibacterial agents, antifungal agents, hypoglycemic agents, antihistamine agents, chemoattractants, neutraceuticals, antiobesity, smoking cessation agents and antiasmatic agents.
81. The method of making an electromatrix device of clause 67, wherein the pharmacologically active agent comprises a second, migration-vulnerable drug delivery device.
82. The method of making an electromatrix device of clause 68, wherein the pharmacologically active agent comprises a second, migration-vulnerable drug delivery device.
83. The method of making an electromatrix device of clause 81, wherein the migration-vulnerable drug delivery device comprises a plurality of lipospheres, microspheres or a combination thereof homogeneously dispersed within the electromatrix device.
84. The method of making an electromatrix device of clause 82, wherein the migration-vulnerable drug delivery device comprises a plurality of lipospheres, microspheres or a combination thereof homogeneously dispersed within the electromatrix device.
85. The method of making an electromatrix device of clause 67, wherein the pharmacologically active agent is substantially homogeneously distributed within the electromatrix device.
86. The method of making an electromatrix device of clause 68, wherein the pharmacologically active agent is substantially homogeneously distributed within the electromatrix device.
87. The method of making an electromatrix device of clause 67 further comprising one or more biocompatible polymeric materials.
88. The method of making an electromatrix device of clause 68 further comprising one or more biocompatible polymeric materials.
89. The method of making an electromatrix device of clause 87 wherein the one or more biocompatible polymeric materials are selected from the group consisting of epoxies, polyesters, acrylics, nylons, silicones, polyanhydride, polyurethane, polycarbonate, poly(tetrafluoroethylene), polycaprolactone, polyethylene oxide, polyethylene glycol, poly(vinyl chloride), polylactic acid, polyglycolic acid, polypropylene oxide, poly(akylene)glycol, polyoxyethylene, sebacic acid, polyvinyl alcohol, 2-hydroxyethyl methacrylate, polymethyl methacrylate,1,3-bis(carboxyphenoxy)propane, lipids, phosphatidylcholine, triglycerides, polyhydroxybutyrate, polyhydroxyvalerate, poly(ethylene oxide), poly ortho esters, poly (amino acids), polycynoacrylates, polyphophazenes, polysulfone, polyamine, poly (amido amines), fibrin, graphite, flexible fluoropolymer, isobutyl-based, isopropyl styrene, vinyl pyrrolidone, cellulose acetate dibutyrate, silicone rubber, and copolymers of these.
90. The method of making an electromatrix device of clause 88 wherein the one or more biocompatible polymeric materials are selected from the group consisting of epoxies, polyesters, acrylics, nylons, silicones, polyanhydride, polyurethane, polycarbonate, poly(tetrafluoroethylene), polycaprolactone, polyethylene oxide, polyethylene glycol, poly(vinyl chloride), polylactic acid, polyglycolic acid, polypropylene oxide, poly(akylene)glycol, polyoxyethylene, sebacic acid, polyvinyl alcohol, 2-hydroxyethyl methacrylate, polymethyl methacrylate, 1,3-bis(carboxyphenoxy)propane, lipids, phosphatidylcholine, triglycerides, polyhydroxybutyrate, polyhydroxyvalerate, poly(ethylene oxide), poly ortho esters, poly (amino acids), polycynoacrylates, polyphophazenes, polysulfone, polyamine, poly (amido amines), fibrin, graphite, flexible fluoropolymer, isobutyl-based, isopropyl styrene, vinyl pyrrolidone, cellulose acetate dibutyrate, silicone rubber, and copolymers of these.
91. The method of making an electromatrix device of clause 67 wherein the current released drug delivery device is crosslinked with one or more crosslinking agents.
92. The method of making an electromatrix device of clause 68 wherein the current released drug delivery device is crosslinked with one or more crosslinking agents.
93. The method of making an electromatrix device of clause 91 wherein the crosslinking agents are selected from the group consisting of glutaraldehyde, p-Azidobenzolyl Hydazide, N-5-Azido-2 nitrobenzoyloxysuccinimide, N-Succinimidyl 6-[4'azido-2'nitro-phenylamino]hexanoate and 4 [p-Azidosalicylamido] butylamine.
94. The method of making an electromatrix device of clause 92 wherein the one or more crosslinking reagents are selected from the group consisting of glutaraldehyde, p-Azidobenzolyl Hydazide, N-5-Azido 2-nitrobenzoyioxysuccinimide, N-Succinimidyl 6-[4'azido-2'nitro-phenylamino]hexanoate and 4-[p-Azidosalicylamido] butylamine.
95. The method of making an electromatrix device of clause 67 wherein the one or more conductive materials are selected from the group consisting of gold, silver, aluminum, platinum, tungsten, stainless steel, nitinol, copper, niobium, titanium, and ceramics.
96. The method of making an electromatrix device of clause 68 wherein the one or more conductive materials are selected from the group consisting of gold, silver, alumimun, platinum, tungsten, stainless steel, nitinol, copper, niobium, titanium, and ceramics.
97. The method of making an electromatrix device of clause 67 wherein the one or more conductive materials comprises an alloy including one or more substances selected from the group consisting of gold, silver, tungsten, niobium, cobalt, titanium, zirconium, vanadium, molybdenum, nickel, iron, zinc, and copper.
98. The method of making an electromatrix device of clause 68 wherein the one or more conductive materials comprises an alloy including one or more substances selected from the group consisting of gold, silver, tungsten, niobium, cobalt, titanium, zirconium, vanadium, molybdenum, nickel, iron, zinc, and copper.
99. A protein matrix coating for an implantable medical device comprising one or more biocompatible protein materials, one or more conductive materials, one or more pharmacologically active agents and one or more biocompatible solvents, wherein the protein materials, conductive materials, pharmacologically active agents and biocompatible solvents are compressed to remove bulk biocompatible solvent and generate additional interactive forces to form the protein matrix coating.
100. The protein matrix coating for an implantable medical device of clause 99 wherein the biocompatible proteins may be natural, synthetic or genetically engineered.
101. The protein matrix coating for an implantable medical device of clause 100 wherein the biocompatible proteins are natural proteins selected from the group consisting of elastin, collagen, albumin, keratin, fibronectin, silk, silk fibroin, actin, myosin, fibrinogen, thrombin, aprotinin and antithrombin III.
102. The protein matrix coating for an implantable medical device of clause 100 wherein the biocompatible proteins are genetically engineered proteins made of blocks selected from the group consisting of elastinlike blocks, silklike blocks, collagenlike blocks, lamininlike blocks, fibronectinlike blocks and silklike and elastinlike blocks.
103. The protein matrix coating for an implantable medical device of Clause 99 wherein the biocompatible solvent is selected from the group consisting of water, dimethyl sulfoxide (DMSO), biocompatible alcohols, biocompatible acids, oils and biocompatible glycols.
104. The protein matrix coating for an implantable medical device of clause 103 wherein the biocompatible solvent is water.
105. The protein matrix coating for an implantable medical device of clause 99 wherein the one or more pharmacologically active agents are selected from the group consisting of analgesics, anesthetics, antipsychotic agents, steroids, antisteroids, corticosteroids, antiglacoma agents, antialcohol agents, anti-coagulants agents, genetic material, antithrombogenic agents, anticancer agents, anti-Parkinson agents, antiepileptic agents, anti-inflammatory agents, anticonception agents, enzymes agents, cells, growth factors, antiviral agents, antibacterial agents, antifungal agents, hypoglycemic agents, antihistamine agents, chemoattractants, neutraceuticals, antiobesity, smoking cessation agents, obstetric agents and antiasmatic agents.
106. The protein matrix coating for an implantable medical device of clause 99, wherein the pharmacologically active agents comprises a second, migration-vulnerable drug delivery device.
107. The protein matrix coating for an implantable medical device of clause 106, wherein the migration-vulnerable drug delivery device comprises a plurality of lipospheres homogeneously dispersed within the protein matrix coating.
108. The protein matrix coating for an implantable medical device of clause 106, wherein the migration-vulnerable drug delivery device comprises a plurality of microspheres homogeneously dispersed within the protein matrix coating.
109. The protein matrix coating for an implantable medical device of clause 99, wherein the pharmacologically active agent is substantially homogeneously distributed within the protein matrix coating.
110. The protein matrix coating for an implantable medical device of clause 99 further comprising one or more biocompatible polymeric materials.
111. The protein matrix coating for an implantable medical device of clause 110 wherein the one or more biocompatible polymeric materials are selected from the group consisting of epoxies, polyesters, acrylics, nylons, silicones, polyanhydride, polyurethane, polycarbonate, poly(tetrafluoroethylene), polycaprolactone, polyethylene oxide, polyethylene glycol, poly(vinyl chloride), polylactic acid, polyglycolic acid, polypropylene oxide, poly(akylene)glycol, polyoxyethylene, sebacic acid, polyvinyl alcohol, 2-hydroxyethyl methacrylate, polymethyl methacrylate, 1,3-bis(carboxyphenoxy)propane, lipids, phosphatidylcholine, triglycerides, polyhydroxybutyrate, polyhydroxyvalerate, poly(ethylene oxide), poly ortho esters, poly (amino acids), polycynoacrylates, polyphophazenes, polysulfone, polyamine, poly (amido amines), fibrin, graphite, flexible fluoropolymer, isobutyl-based, isopropyl styrene, vinyl pyrrolidone, cellulose acetate dibutyrate, silicone rubber, and copolymers of these.
112. The protein matrix coating for an implantable medical device of clause 99 wherein the current released drug delivery device is crosslinked with one or more crosslinking agents.
113. The protein matrix coating for an implantable medical device of clause 112 wherein the one or more crosslinking reagents are selected from the group consisting of glutaraldehyde, p-Azidobenzolyl Hydazide, N-5-Azido 2-nitrobenzoyloxysuccinimide, N-Succinimidyl 6-[4'azido-2'nitro-phenylamino]hexanoate and 4-[p-Azidosalicylamido] butylamine.
114. The protein matrix coating for an implantable medical device of clause 99 wherein the one or more conductive materials are selected from the group consisting of gold, silver, aluminum, platinum, tungsten, stainless steel, nitinol, copper, niobium, titanium, and ceramics.
115. The protein matrix coating for an implantable medical device of clause 99 wherein the one or more conductive materials comprises an alloy including one or more substances selected from the group consisting of gold, silver, tungsten, niobium, cobalt, titanium, zirconium, vanadium, molybdenum, nickel, iron, zinc, and copper.
116. A method of making a protein matrix coating for an implantable medical device, comprising the steps of:
   (a) preparing a coatable composition including the one or more biocompatible protein materials, zero or more pharmacologically active agents and the one or more biocompatible solvents;
   (b) coating the composition to form a film;
   (c) partially drying the coated film until the coated film can be formed into a cohesive body;
   (d) forming said cohesive body;
   (e) adding an implantable medical device to the cohesive body; and
   (f) compressing the cohesive body and the medical device to form a protein matrix coating around the medical device.
117. The method of making a protein matrix coating for an implantable medical device of clause 116 wherein the biocompatible proteins may be natural, synthetic or genetically engineered.
118. The method of making a current released drug delivery device of clause 19 wherein the biocompatible proteins may be natural, synthetic or genetically engineered.
119. The method of making a protein matrix coating for an implantable medical device of clause 118 wherein the biocompatible proteins are natural proteins selected from the group consisting of elastin, collagen, albumin, keratin, fibronectin, silk, silk fibroin, actin, myosin, fibrinogen, thrombin, aprotinin and antithrombin Ill.
120. The method of making a protein matrix coating for an implantable medical device of clause 118 wherein the biocompatible proteins are genetically engineered proteins made of blocks selected from the group consisting of elastinlike blocks, silklike blocks, collagenlike blocks, lamininlike blocks, fibronectinlike blocks and silklike and elastinlike blocks.
121. The method of making a protein matrix coating for an implantable medical device of clause 116 wherein the biocompatible solvent is selected from the group consisting of water, dimethyl sulfoxide (DMSO), biocompatible alcohols, biocompatible acids, oils and biocompatible glycols.
122. The method of making a current released drug delivery device of clause 121 wherein the biocompatible solvent is water.
123. The method of making a protein matrix coating for an implantable medical device of clause 116 wherein the one or more pharmacologically active agents are selected from the group consisting of analgesics, anesthetics, anti psychotic agents, steroids, antisteroids, corticosteroids, antiglacoma agents, antialcohol agents, anticoagulants agents, genetic material, antithrombolytic agents, anticancer agents, anti-Parkinson agents, antiepileptic agents, anti-inflammatory agents, anticonception agents, enzymes agents, cells, growth factors, antiviral agents, antibacterial agents, antifungal agents, hypoglycemic agents, antihistamine agents, chemoattractants, neutraceuticals, antiobesity, smoking cessation agents and antiasmatic agents.
124. The method of making a protein matrix coating for an implantable medical device of clause 116, wherein the pharmacologically active agent comprises a second, migration-vulnerable drug delivery device.
125. The method of making a protein matrix coating for an implantable medical device of clause 124, wherein the migration-vulnerable drug delivery device comprises a plurality of lipospheres, microspheres or a combination thereof homogeneously dispersed within the protein matrix coating.
126. The method of making a protein matrix coating for an implantable medical device of clause 116, wherein the pharmacologically active agent is substantially homogeneously distributed within the protein matrix coating.
127. The method of making a protein matrix coating for an implantable medical device of clause 116 further comprising one or more biocompatible polymeric materials.
128. The method of making a protein matrix coating for an implantable medical device of clause 127 wherein the one or more biocompatible polymeric materials are selected from the group consisting of epoxies, polyesters, acrylics, nylons, silicones, polyanhydride, polyurethane, polycarbonate, poly(tetrafluoroethylene), polycaprolactone, polyethylene oxide, polyethylene glycol, poly(vinyl chloride), polylactic acid, polyglycolic acid, polypropylene oxide, poly(akylene)glycol, polyoxyethylene, sebacic acid, polyvinyl alcohol, 2-hydroxyethyl methacrylate, polymethyl methacrylate, 1,3-bis(carboxyphenoxy)propane, lipids, phosphatidylcholine, triglycerides, polyhydroxybutyrate, polyhydroxyvalerate, poly(ethylene oxide), poly ortho esters, poly (amino acids), polycynoacrylates, polyphophazenes, polysulfone, polyamine, poly (amido amines), fibrin, graphite, flexible fluoropolymer, isobutyl-based, isopropyl styrene, vinyl pyrrolidone, cellulose acetate dibutyrate, silicone rubber, and copolymers of these.
129. The method of making a protein matrix coating for an implantable medical device of clause 116 wherein the protein matrix material is crosslinked with one or more crosslinking agents.
130. The method of making a protein matrix coating for an implantable medical device of clause 129 wherein the crosslinking agents are selected from the group consisting of glutaraldehyde, p-Azidobenzolyl Hydazide, N-5-Azido-2 nitrobenzoyloxysuccinimide, N-Succinimidyl 6-[4'azido-2'nitro-phenylamino]hexanoate and 4 [p-Azidosalicylamido] butylamine.
131. The method of making a protein matrix coating for an implantable medical device of clause 116 wherein the one or more.conductive materials are selected from the group consisting of gold, silver, aluminum, platinum, tungsten, stainless steel, nitinol, copper, niobium, titanium, and ceramics.
132. The method of making a protein matrix coating for an implantable medical device of clause 116 wherein the one or more conductive materials comprises an alloy including one or more substances selected from the group consisting of gold, silver, tungsten, niobium, platinum cobalt, titanium, zirconium, vanadium, molybdenum, nickel, iron, zinc, and copper.
133. A protein matrix array comprising one or more biocompatible protein materials, zero or more pharmacologically active agents and one or more biocompatible solvents, wherein the protein materials, pharmacologically active agents and biocompatible solvents are compressed to remove bulk biocompatible solvent and generate additional interactive forces to form the protein matrix array.
134. A method of making a protein matrix array, comprising the steps of:
   (a) preparing a coatable composition comprising one or more biocompatible protein materials, zero or more pharmacologically active agents and one or more biocompatible solvents;
   (b) coating the composition to form a film;
   (c) partially drying the coated film until the coated film can be formed into a cohesive body;
   (d) forming said cohesive body; and compressing the cohesive body to form a protein matrix array.
135. A method of determining pharmacologically active agent to protein interaction comprising:
   (a) introducing a pharmacologically active agent to a protein matrix array comprising one or more biocompatible protein materials, zero or more pharmacologically active agents and one or more biocompatible solvents, wherein the protein materials, pharmacologically active agents and biocompatible solvents are compressed to remove bulk biocompatible solvent and generate additional interactive forces to form the protein matrix array;
   (b) testing for pharmacologically active agent to protein interaction.

## Claims

1. A device comprising one or more biocompatible protein matrix materials, the protein matrix materials including one or more biocompatible proteins and one or more biocompatible solvents, wherein the biocompatible proteins and biocompatible solvents are formed into a cohesive body having a solvent content of about 20% to 80% prior to compression and the cohesive body is compressed at a pressure of about 689 kPa to 689,500 kPa (100 psi to 100,000 psi) to remove bulk biocompatible solvent and generate additional intermolecular and intramolecular forces between the one or more biocompatible proteins and solvents to form the device having a solvent content of about 10% to 60%, wherein the device a protein matrix diagnostic array the protein matrix material is formed into a plate and has one or more indication sites;
a protein matrix coated implantable medical device comprising, a medical device coated with the protein matrix material; or
an electromatrix device.

2. The device of Claim 1, wherein the device is a protein matrix diagnostic array and the protein matrix
materials cover a substrate.

3. A method of determining pharmacologically active agent to protein interaction comprising:
(a) introducing a pharmacologically active agent to a protein matrix diagnostic array of claim 1 or 2, , the protein matrix array comprising one or more biocompatible protein matrix materials formed into a plate and having one or more indication sites, the protein matrix materials including one or more biocompatible proteins and one or more biocompatible solvents, wherein the biocompatible proteins and biocompatible solvents are formed into a cohesive body having a solvent content of about 20% to 80% prior to compression and the cohesive body is compressed at a pressure of about 689 kPa to 689,500 kPa (100 psi to 100,000 psi) to remove bulk biocompatible solvent and generate additional intermolecular and intramolecular forces between the one or more biocompatible proteins and solvents to form the protein matrix diagnostic array having a solvent content of about 10% to 60%;
(b) testing for pharmacologically active agent to protein interaction.

4. A method of making the device of claim 1, comprising the steps of:
(a) preparing a cohesive body including the one or more biocompatible proteins and the one or more biocompatible solvents, the cohesive body having a solvent content of about 20% to 80% prior to compression; and
(b) compressing the cohesive body at a pressure of about 689 kPa to 689,500 kPa (100 psi to 100,000 psi) to remove bulk biocompatible solvent and generate additional intermolecular and intramolecular forces between one or more of the protein materials, conductive materials, active agents and solvents to form a device having a solvent content of about 10% to 60%.

5. The method of claim 4, wherein the device is a protein matrix coated implantable medical device and step (a) further comprises adding an implantable medical device to the cohesive body.

6. The device of claim 1 or 2 or the method of claims 3, 4 or 5, wherein the biocompatible proteins are natural, synthetic or genetically engineered.

7. The device or method of claim 6, wherein the biocompatible proteins are natural proteins selected from the group consisting of elastin, collagen, albumin, keratin, fibronectin, silk, silk fibroin, actin, myosin, fibrinogen, thrombin, aprotinin and antithrombin III.

8. The device or method of Claim 6 wherein the biocompatible proteins aregenetically engineered proteins made of blocks selected from the group consisting of elastinlike blocks, silklike blocks, collagenlike blocks, lamininlike blocks, fibronectinlike blocks and silklike and elastinlike blocks.

9. The device of Claim 1, 2 or 6 to 8, or the method of any one of Claims 3to 8 wherein the biocompatible solvent is selected from the group consisting of water, dimethyl sulfoxide (DMSO), biocompatible alcohols, biocompatible acids, oils and biocompatible glycols, preferably water.

10. The protein matrix diagnostic array of Claim 1, 2 or 6 to 9, or the method of Claim 3 to 9 including one or more pharmacologically active agents selected from the group consisting of analgesics, anesthetics, antipsychotic agents, steroids, antisteroids, cortisteroids, antiglaucoma agents, antialcohol agents, anti-coagulants agents, genetic material, antithrombogenic agents, antithrombolytic agents, anticancer agents, anti-Parkinson agents, antiepileptic agents, anti-inflammatory agents, anticonception agents, enzymes agents, cells, growth factors, antiviral agents, antibacterial agents, antifungal agents, hypoglycemic agents, antihistamine agents, chemoattractants, neutraceuticals, antiobesity, smoking cessation agents, obstetric agents and antiasthmatic agents.

11. The protein matrix diagnostic array of Claim 1, 2 or 6 to 10, or the method of Claim 3 to 10, wherein the pharmacologically active agent comprises a second, migration-vulnerable drug delivery device.

12. The deviceor method of Claim 11, wherein the migration-vulnerable drug delivery device comprises a plurality of lipospheres, microspheres or a combination thereof homogeneously dispersed within the drug delivery device, the electromatrix device or the protein matrix coating

13. The device of Claim 1, 2 or 6 to 12, or the method of Claim 3 to 12, further comprising one or more biocompatible polymeric materials,.
selected from the group consisting of epoxies, polyester, acrylics, nylons, silicones, polyanhydride, polyurethane, polycarbonate, poly(tetrafluoroethylene), polycaprolactone, polyethylene oxide, polyethylene glycol, polyvinyl chloride), polylactic acid, polyglycolic acid, polypropylene oxide, poly(akylene)glycol, polyoxyethylene, sebacic acid, polyvinyl alcohol, 2-hydroxyethyl methacrylate, polymethyl methacrylate, 1,3- bis(carboxyphenoxy)propane, lipids, phosphatidylcholine, triglycerides, polyhydroxybuyrate, polyhydroxyvalerate, poly(ethylene oxide), poly ortho esters, poly (amino acids), polycynoacrylates, polyphophazenes, polysulfone, polyamine, poly (amido amines), fibrin, graphite, flexible fluoropolymer, isobutyl-based, isopropyl styrene, vinyl pyrrolidone, cellulose acetate dibutyrate, silicone rubber, and copolymers of these.

14. The device of Claim 1, 2 or 6 to 13, or the method of Claim 3to 13, wherein the protein matrix is crosslinked with one or more crosslinking agents selected from the group consisting of glutaraldehyde, p-Azidobenzolyl Hydazide, N- 5-Azido 2 nitrobenzoyloxysuccinimide, N-Succinimidyl 6-[4'azido-2'nitro phenylamino]hexanoate and 4-[p-Azidosalicylamido] butylamine.

15. The device of Claim 1, 2 or 6 to 14, or the method of Claim 3 to 14 wherein the protein matrix further comprises one or more conductive materials selected from the group consisting of gold, silver, aluminum, platinum, tungsten, stainless steel, nitinol, copper, niobium, titanium, ceramics,
and an alloy including one or more substances selected from the group consisting of gold, silver, tungsten, niobium, platinum, cobalt, titanium, zirconium, vanadium, molybdenum, nickel, iron, zinc, and copper.


SEQUENCE LISTING

<110>  Masters, David B.

<120>  Device Including Protein Matrix Materials And Methods Of Making And Using
Same

<130>  45795.23.1

<140>  US 09/922,418
<141>  2001-08-03

<160>  21

<170>  PatentIn version 3.3

<210>  1
<211>  59
<212>  PRT
<213>  Artificial

<220>
<223>  synthetic construct similar to silk protein

<400>  1

Gly Ala Gly Ala Gly Ser Gly Ala Gly Ala Gly Ser Gly Ala Gly Ala
1               5                   10                  15

Gly Ser Gly Ala Gly Ala Gly Ser Gly Ala Gly Ala Gly Ser Gly Ala
            20                  25                  30

Gly Ala Gly Ser Gly Ala Gly Ala Gly Ser Gly Ala Gly Ala Gly Ser
        35                  40                  45

Gly Ala Gly Ala Gly Ser Gly Ala Ala Gly Tyr
    50                  55

<210>  2
<211>  6
<212>  PRT
<213>  Artificial

<220>
<223>  seq. repeated indefinitely,  synthetic construct similar to silk protein

<400>  2

Gly Ala Gly Ala Gly Ser
1               5

<210>  3
<211>  71
<212>  PRT
<213>  Artificial

<220>
<223>  seq. repeated indefinitely, synthetic construct similar to silk protein
containing RGD sequence from fibronectin.

<400>  3

Gly Ala Gly Ala Gly Ser Gly Ala Gly Ala Gly Ser Gly Ala Gly Ala

1               5                   10                  15

Gly Ser Gly Ala Gly Ala Gly Ser Gly Ala Gly Ala Gly Ser Gly Ala
            20                  25                  30

Gly Ala Gly Ser Gly Ala Gly Ala Gly Ser Gly Ala Gly Ala Gly Ser
        35                  40                  45

Gly Ala Gly Ala Gly Ser Gly Ala Ala Val Thr Gly Arg Gly Asp Ser
    50                  55                  60

Pro Ala Ser Ala Ala Gly Tyr
65                  70

<210>  4
<211>  74
<212>  PRT
<213>  Artificial

<220>
<223>  seq. repeated indefinitely, synthetic construct similar to silk protein
containing sequence from laminin protein.

<400>  4

Gly Ala Gly Ala Gly Ser Gly Ala Gly Ala Gly Ser Gly Ala Gly Ala
1               5                   10                  15

Gly Ser Gly Ala Gly Ala Gly Ser Gly Ala Gly Ala Gly Ser Gly Ala
            20                  25                  30

Gly Ala Gly Ser Gly Ala Gly Ala Gly Ser Gly Ala Gly Ala Gly Ser
        35                  40                  45

Gly Ala Gly Ala Gly Ser Gly Ala Ala Pro Gly Ala Ser Ile Lys Val
    50                  55                  60

Ala Val Ser Ala Gly Pro Ser Ala Gly Tyr
65                  70

<210>  5
<211>  73
<212>  PRT
<213>  Artificial

<220>
<223>  seq. repeated indefinitely, synthetic construct similar to silk protein
containing a different sequence from laminin protein.

<400>  5

Gly Ala Gly Ala Gly Ser Gly Ala Gly Ala Gly Ser Gly Ala Gly Ala
1               5                   10                  15

Gly Ser Gly Ala Gly Ala Gly Ser Gly Ala Gly Ala Gly Ser Gly Ala
            20                  25                  30

Gly Ala Gly Ser Gly Ala Gly Ala Gly Ser Gly Ala Gly Ala Gly Ser
        35                  40                  45

Gly Ala Gly Ala Gly Ser Gly Ala Ala Pro Gly Ala Ser Ile Lys Val
    50                  55                  60

Ala Val Ser Gly Pro Ser Ala Gly Tyr
65                  70

<210>  6
<211>  71
<212>  PRT
<213>  Artificial

<220>
<223>  seq. repeated indefinitely, synthetic construct similar to silk protein
containing the RGD sequence from fibronectin.

<400>  6

Gly Ala Gly Ala Gly Ser Gly Ala Gly Ala Gly Ser Gly Ala Gly Ala
1               5                   10                  15

Gly Ser Gly Ala Gly Ala Gly Ser Gly Ala Gly Ala Gly Ser Gly Ala
            20                  25                  30

Gly Ala Gly Ser Gly Ala Gly Ala Gly Ser Gly Ala Gly Ala Gly Ser
        35                  40                  45

Gly Ala Gly Ala Gly Ser Arg Tyr Val Val Leu Pro Arg Pro Val Cys
    50                  55                  60

Phe Glu Lys Ala Ala Gly Tyr
65                  70

<210>  7
<211>  20
<212>  PRT
<213>  Artificial

<220>
<223>  seq. repeated indefinitely, synthetic construct similar to elastin
protein.

<400>  7

Val Pro Gly Val Gly Val Pro Gly Val Gly Val Pro Gly Val Gly Val
1               5                   10                  15

Pro Gly Val Gly
            20

<210>  8
<211>  52
<212>  PRT
<213>  Artificial

<220>
<223>  seq. repeated indefinitely, synthetic construct similar to silk and
elastin proteins.

<400>  8

Gly Val Gly Val Pro Gly Val Gly Val Pro Gly Val Gly Val Pro Gly
1               5                   10                  15

Val Gly Val Pro Gly Val Gly Val Pro Gly Val Gly Val Pro Gly Val
            20                  25                  30

Gly Val Pro Gly Val Gly Val Pro Gly Ala Gly Ala Gly Ser Gly Ala
        35                  40                  45

Gly Ala Gly Ser
    50

<210>  9
<211>  82
<212>  PRT
<213>  Artificial

<220>
<223>  seq. repeated indefinitely, synthetic construct similar to silk and
elastin proteins.

<400>  9

Gly Ala Ala Val Pro Gly Val Gly Val Pro Gly Val Gly Val Pro Gly
1               5                   10                  15

Val Gly Val Pro Gly Val Gly Val Ala Ala Gly Tyr Gly Ala Gly Ala
            20                  25                  30

Gly Ser Gly Ala Gly Ala Gly Ser Gly Ala Gly Ala Gly Ser Gly Ala
        35                  40                  45

Gly Ala Gly Ser Gly Ala Gly Ala Gly Ser Gly Ala Gly Ala Gly Ser
    50                  55                  60

Gly Ala Gly Ala Gly Ser Gly Ala Gly Ala Gly Ser Gly Ala Gly Ala
65                  70                  75                  80

Gly Ser

<210>  10
<211>  111
<212>  PRT
<213>  Artificial

<220>
<223>  seq. repeated indefinitely, synthetic construct similar to silk and
elastin proteins.

<400>  10

Gly Ala Gly Ala Gly Ser Gly Ala Gly Ala Gly Ser Gly Ala Gly Ala
1               5                   10                  15

Gly Ser Gly Ala Gly Ala Gly Ser Gly Ala Gly Ala Gly Ser Gly Ala
            20                  25                  30

Gly Ala Gly Ser Gly Ala Ala Gly Tyr Gly Ala Gly Ala Gly Ser Gly
        35                  40                  45

Ala Gly Ala Gly Ser Gly Ala Gly Ala Gly Ser Gly Ala Gly Ala Gly
    50                  55                  60

Ser Gly Ala Gly Ala Gly Ser Gly Val Gly Val Pro Gly Val Gly Val
65                  70                  75                  80

Pro Gly Val Gly Val Pro Gly Val Gly Val Pro Gly Val Gly Val Pro
                85                  90                  95

Gly Val Gly Val Pro Gly Val Gly Val Pro Gly Val Gly Val Pro
            100                 105                 110

<210>  11
<211>  88
<212>  PRT
<213>  Artificial

<220>
<223>  seq. repeated indefinitely, synthetic construct similar to silk and
elastin proteins.

<400>  11

Gly Val Gly Val Pro Gly Val Gly Val Pro Gly Val Gly Val Pro Gly
1               5                   10                  15

Val Gly Val Pro Gly Val Gly Val Pro Gly Val Gly Val Pro Gly Val
            20                  25                  30

Gly Val Pro Gly Val Gly Val Pro Gly Ala Gly Ala Gly Ser Gly Ala
        35                  40                  45

Gly Ala Gly Ser Gly Ala Gly Ala Gly Ser Gly Ala Gly Ala Gly Ser
    50                  55                  60

Gly Ala Gly Ala Gly Ser Gly Ala Gly Ala Gly Ser Gly Ala Gly Ala
65                  70                  75                  80

Gly Ser Gly Ala Gly Ala Gly Ser
                85

<210>  12
<211>  108
<212>  PRT
<213>  Artificial

<220>
<223>  seq. repeated indefinitely, synthetic construct similar to silk and
elastin proteins.

<400>  12

Gly Val Gly Val Pro Gly Val Gly Val Pro Gly Val Gly Val Pro Gly
1               5                   10                  15

Val Gly Val Pro Gly Val Gly Val Pro Gly Val Gly Val Pro Gly Val
            20                  25                  30

Gly Val Pro Gly Val Gly Val Pro Gly Val Gly Val Pro Gly Val Gly
        35                  40                  45

Val Pro Gly Val Gly Val Pro Gly Val Gly Val Pro Gly Ala Gly Ala
    50                  55                  60

Gly Ser Gly Ala Gly Ala Gly Ser Gly Ala Gly Ala Gly Ser Gly Ala
65                  70                  75                  80

Gly Ala Gly Ser Gly Ala Gly Ala Gly Ser Gly Ala Gly Ala Gly Ser
                85                  90                  95

Gly Ala Gly Ala Gly Ser Gly Ala Gly Ala Gly Ser
            100                 105

<210>  13
<211>  128
<212>  PRT
<213>  Artificial

<220>
<223>  seq. repeated indefinitely, synthetic construct similar to silk and
elastin proteins.

<400>  13

Gly Val Gly Val Pro Gly Val Gly Val Pro Gly Val Gly Val Pro Gly
1               5                   10                  15

Val Gly Val Pro Gly Val Gly Val Pro Gly Val Gly Val Pro Gly Val
            20                  25                  30

Gly Val Pro Gly Val Gly Val Pro Gly Val Gly Val Pro Gly Val Gly
        35                  40                  45

Val Pro Gly Val Gly Val Pro Gly Val Gly Val Pro Gly Val Gly Val
    50                  55                  60

Pro Gly Val Gly Val Pro Gly Val Gly Val Pro Gly Val Gly Val Pro
65                  70                  75                  80

Gly Ala Gly Ala Gly Ser Gly Ala Gly Ala Gly Ser Gly Ala Gly Ala
                85                  90                  95

Gly Ser Gly Ala Gly Ala Gly Ser Gly Ala Gly Ala Gly Ser Gly Ala
            100                 105                 110

Gly Ala Gly Ser Gly Ala Gly Ala Gly Ser Gly Ala Gly Ala Gly Ser
        115                 120                 125

<210>  14
<211>  208
<212>  PRT
<213>  Artificial

<220>
<223>  seq. repeated indefinitely, synthetic construct similar to silk and
elastin proteins.

<400>  14

Gly Val Gly Val Pro Gly Val Gly Val Pro Gly Val Gly Val Pro Gly
1               5                   10                  15

Val Gly Val Pro Gly Val Gly Val Pro Gly Val Gly Val Pro Gly Val
            20                  25                  30

Gly Val Pro Gly Val Gly Val Pro Gly Val Gly Val Pro Gly Val Gly
        35                  40                  45

Val Pro Gly Val Gly Val Pro Gly Val Gly Val Pro Gly Val Gly Val
    50                  55                  60

Pro Gly Val Gly Val Pro Gly Val Gly Val Pro Gly Val Gly Val Pro
65                  70                  75                  80

Gly Val Gly Val Pro Gly Val Gly Val Pro Gly Val Gly Val Pro Gly
                85                  90                  95

Val Gly Val Pro Gly Val Gly Val Pro Gly Val Gly Val Pro Gly Val
            100                 105                 110

Gly Val Pro Gly Val Gly Val Pro Gly Val Gly Val Pro Gly Val Gly
        115                 120                 125

Val Pro Gly Val Gly Val Pro Gly Val Gly Val Pro Gly Val Gly Val
    130                 135                 140

Pro Gly Val Gly Val Pro Gly Val Gly Val Pro Gly Val Gly Val Pro
145                 150                 155                 160

Gly Ala Gly Ala Gly Ser Gly Ala Gly Ala Gly Ser Gly Ala Gly Ala
                165                 170                 175

Gly Ser Gly Ala Gly Ala Gly Ser Gly Ala Gly Ala Gly Ser Gly Ala
            180                 185                 190

Gly Ala Gly Ser Gly Ala Gly Ala Gly Ser Gly Ala Gly Ala Gly Ser
        195                 200                 205

<210>  15
<211>  76
<212>  PRT
<213>  Artificial

<220>
<223>  seq. repeated indefinitely, synthetic construct similar to silk and
elastin proteins.

<400>  15

Gly Val Gly Val Pro Gly Val Gly Val Pro Gly Val Gly Val Pro Gly
1               5                   10                  15

Val Gly Val Pro Gly Val Gly Val Pro Gly Val Gly Val Pro Gly Val
            20                  25                  30

Gly Val Pro Gly Val Gly Val Pro Gly Ala Gly Ala Gly Ser Gly Ala
        35                  40                  45

Gly Ala Gly Ser Gly Ala Gly Ala Gly Ser Gly Ala Gly Ala Gly Ser
    50                  55                  60

Gly Ala Gly Ala Gly Ser Gly Ala Gly Ala Gly Ser
65                  70                  75

<210>  16
<211>  64
<212>  PRT
<213>  Artificial

<220>
<223>  seq. repeated indefinitely, synthetic construct similar to silk and
elastin proteins.

<400>  16

Gly Val Gly Val Pro Gly Val Gly Val Pro Gly Val Gly Val Pro Gly
1               5                   10                  15

Val Gly Val Pro Gly Val Gly Val Pro Gly Val Gly Val Pro Gly Val
            20                  25                  30

Gly Val Pro Gly Val Gly Val Pro Gly Ala Gly Ala Gly Ser Gly Ala
        35                  40                  45

Gly Ala Gly Ser Gly Ala Gly Ala Gly Ser Gly Ala Gly Ala Gly Ser
    50                  55                  60

<210>  17
<211>  56
<212>  PRT
<213>  Artificial

<220>

<223>  seq. repeated indefinitely, synthetic construct similar to keratin
protein.

<400>  17

Ala Lys Leu Lys Leu Ala Glu Ala Lys Leu Glu Leu Ala Glu Ala Lys
1               5                   10                  15

Leu Lys Leu Ala Glu Ala Lys Leu Glu Leu Ala Glu Ala Lys Leu Lys
            20                  25                  30

Leu Ala Glu Ala Lys Leu Glu Leu Ala Glu Ala Lys Leu Lys Leu Ala
        35                  40                  45

Glu Ala Lys Leu Glu Leu Ala Glu
    50                  55

<210>  18
<211>  15
<212>  PRT
<213>  Artificial

<220>
<223>  seq. repeated indefinitely, synthetic construct similar to collagen
protein.

<400>  18

Gly Ala Pro Gly Pro Pro Gly Pro Pro Gly Pro Pro Gly Pro Pro
1               5                   10                  15

<210>  19
<211>  39
<212>  PRT
<213>  Artificial

<220>
<223>  seq. repeated indefinitely,  synthetic construct similar to collagen
protein.

<400>  19

Gly Ala Pro Gly Pro Pro Gly Pro Pro Gly Pro Pro Gly Pro Pro Gly
1               5                   10                  15

Ala Pro Gly Pro Pro Gly Pro Pro Gly Pro Pro Gly Pro Pro Gly Pro
            20                  25                  30

Ala Gly Pro Val Gly Ser Pro
        35

<210>  20
<211>  63
<212>  PRT
<213>  Artificial

<220>
<223>  seq. repeated indefinitely, synthetic construct similar to collagen
protein with a cell binding domain from human collagen.

<400>  20

Gly Ala Pro Gly Pro Pro Gly Pro Pro Gly Pro Pro Gly Pro Pro Gly
1               5                   10                  15

Ala Pro Gly Pro Pro Gly Pro Pro Gly Pro Pro Gly Pro Pro Gly Leu
            20                  25                  30

Pro Gly Pro Lys Gly Asp Arg Gly Asp Ala Gly Pro Lys Gly Ala Asp
        35                  40                  45

Gly Ser Pro Gly Pro Ala Gly Pro Ala Gly Pro Val Gly Ser Pro
    50                  55                  60

<210>  21
<211>  15
<212>  PRT
<213>  Artificial

<220>
<223>  seq. repeated indefinitely, synthetic construct similar to collagen
protein.

<400>  21

Gly Ala Pro Gly Ala Pro Gly Ser Gln Gly Ala Pro Gly Leu Gln
1               5                   10                  15